(19) Europäisches Patentamt
European Patent Office
Office européen des brevets

(11) Numéro de publication: **0 557 171 B1**

# (12) FASCICULE DE BREVET EUROPEEN

(45) Date de publication de fascicule du brevet: **19.07.95**

(51) Int. Cl.6: **C07D 471/04**, A61K 31/44, G01N 27/416

(21) Numéro de dépôt: **93400366.6**

(22) Date de dépôt: **12.02.93**

## (54) Azaindoles, procédés de préparation et médicaments les contenant.

(30) Priorité: **14.02.92 FR 9201701**

(43) Date de publication de la demande: **25.08.93 Bulletin 93/34**

(45) Mention de la délivrance du brevet: **19.07.95 Bulletin 95/29**

(84) Etats contractants désignés: **AT BE CH DE DK ES FR GB GR IE IT LI LU NL PT SE**

(56) Documents cités:
**EP-A- 0 265 640**
**EP-A- 0 279 263**
**EP-A- 0 415 413**
**EP-A- 0 465 970**
**EP-A- 0 506 532**
**WO-A-91/04027**

(73) Titulaire: **LIPHA, LYONNAISE INDUSTRIELLE PHARMACEUTIOUE
34, rue Saint Romain
Boîte Postale 8481
F-69008 Lyon (FR)**

(72) Inventeur: **Festal, Didier
Pins 5,
Charrièrre Blanche
F-69130 Ecully (FR)**
Inventeur: **Descours, Denis
65, rue Anatole France
F-69100 Villeurbanne (FR)**
Inventeur: **Decerprit, Jacques
83, Chemin de Sermenaz
F-01700 Miribel (FR)**

(74) Mandataire: **Le Guen, Gérard et al
CABINET LAVOIX
2, place d'Estienne d'Orves
F-75441 Paris Cédex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux composés azaindoliques, les procédés de préparation de ces composés, les compositions pharmaceutiques les contenant et leur utilisation comme médicaments notamment dans le traitement des hyperlipidémies et de l'athérosclérose.

On sait que les dépôts lipidiques, notamment les dépôts de cholestérol dans les vaisseaux sont à l'origine de la formation de plaques d'athérome qui sont la cause de maladies cardiovasculaires variées; plus précisémment l'athérome est une forme d'athérosclérose caractérisée par une accumulation excessive de lipides en particulier d'esters du cholestérol dans la paroi des vaisseaux; on a récemment trouvé qu'une enzyme, l'acyl coenzyme A : cholesterol acyl transférase (A.C.A.T.) était responsable de l'estérification du cholestérol, et on a mis en évidence une corrélation entre l'augmentation de l'activité de cette enzyme et l'accumulation d'esters dans la paroi vasculaire; on sait aussi que le cholestérol alimentaire est absorbé sous forme libre puis est estérifié par l'A.C.A.T. intestinale pour être libéré dans la circulation sanguine sous forme de VLDL et/ou de chylomicrons.

On a cherché à mettre au point des produits inhibiteurs de l'A.C.A.T., capables d'empêcher l'absorption intestinale du cholestérol alimentaire et biliaire et de s'opposer aux dépôts d'esters du cholestérol dans la paroi des vaisseaux.

EP 506 532 est un document intercalaire concernant des dérivés de l'indole de formule :

Z-CH2-N(R5)-CO-NH-R6 ,
R4
R2
N
R3
R1
1

aux propriétés hypolipidemiantes, anti-athéromateuses et anti-diabétiques.

WO 9 104 027 décrit des inhibiteurs de l'acyl coenzyme A : cholesterol acyltransferase (ACAT) de formule $R_1N(H)COQ$ lesquels ne présentent pas le noyau azaindole des composés de l'invention.

Cette recherche d'inhibiteurs de l'A.C.A.T. a conduit les inventeurs à élaborer une nouvelle famille d'azaindoles et à trouver que ces produits, de manière inattendue, manifestent une puissante activité inhibitrice de l'A.C.A.T. vasculaire et intestinale associée à un effet anti-hyperlipidémiant intense sur différentes espèces animales.

Ces propriétés des composés de l'invention les rendent particulièrement utiles notamment pour le traitement des hyperlipidémies et de l'athérosclérose.

L'invention concerne plus particulièrement les composés de formule **1** ci-dessous dans laquelle,

C(R5)(R6)-CH(R7)-N(R8)-CO-NHR9

X1
X2
R4
X3
X4
R3

R2
N
R1

**1**

dans laquelle

l'un des groupes $X_1$ à $X_4$ désigne un atome d'azote et les autres représentent un radical CH;

$R_1$ et $R_2$, qui peuvent être situés en position -1, -2 ou -3 du cycle azaindole, représentent chacun un atome d'hydrogène, un radical alkyle linéaire ou ramifié en $C_1$ à $C_{12}$, alcényle en $C_2$ à $C_6$, cycloalkyle ou

cycloalcényle en $C_3$ à $C_8$, alkylthio en $C_1$ à $C_6$ ou lorsqu'ils sont en position -2 ou -3 du azaindole, un atome d'halogène; phényle ou benzyle non substitués ou portant un à trois substituants choisis parmi halogène et les radicaux alkyle, alkoxy et alkylthio en $C_1$ à $C_4$;

$R_3$ et $R_4$ qui peuvent être situés sur les sommets -4, -5, -6 ou -7 du cycle azaindole à condition que ceux-ci figurent un atome de carbone, représentent chacun un atome d'hydrogène ou d'halogène, alkyle, alkoxy ou alkylthio en $C_1$ à $C_6$ ou cycloalkyle en $C_3$ à $C_8$;

le groupement $-C(R_5)(R_6)-CH(R_7)-N(R_8)-CO-NH-R_9$ peut être lié aux sommets -1, -2 ou -3 du cycle azaindole, étant entendu que lorsque l'atome d'azote en position -1 du cycle azaindole n'est pas substitué par l'un des groupes $R_1$, $R_2$ ou $-C(R_5)(R_6)-CH(R_7)-N(R_8)CONHR_9$, il porte un atome d'hydrogène;

$R_5$ et $R_6$ représentent chacun un atome d'hydrogène, un radical alkyle en $C_1$ à $C_{12}$, alcényle en $C_2$ à $C_6$, cycloalkyle ou cycloalcényle en $C_3$ à $C_8$, alkoxyalkyle ou alkylthioalkyle en $C_2$ à $C_{12}$, ou bien

$R_5$ et $R_6$ forment ensemble une chaîne alkylène de formule $-(CH_2)_p-$ éventuellement substitué par un ou deux radicaux alkyle en $C_1$ à $C_4$ et dans laquelle p peut prendre les valeurs 3 à 7, ou une chaîne alkyloxyalkylène de formule $-(CH_2)_x-O-(CH_2)-_y$, dans laquelle x et y peuvent chacun prendre les valeurs 1 ou 2;

$R_7$ désigne un atome d'hydrogène, un radical alkyle en $C_1$ à $C_6$ ou cycloalkyle en $C_3$ à $C_8$;

l'un des substituants $R_5$ à $R_7$ peut aussi représenter un radical phényle ou benzyle non substitués ou portant un à trois substituants choisis parmi halogène et les radicaux alkyle, alkoxy et alkylthio en $C_1$ à $C_4$;

$R_8$ désigne un atome d'hydrogène, un radical alkyle linéaire ou ramifié en $C_1$ à $C_{12}$, un radical cycloalkyle en $C_3$ à $C_8$ ou un radical benzyle non substitué ou portant 1 à 3 substituants choisis parmi halogène et les radicaux alkyle, alkoxy ou alkylthio en $C_1$ à $C_4$;

$R_9$ désigne un radical phényle non substitué ou portant 1 à 3 substituants choisis parmi halogène et les radicaux alkyle, alkoxy et alkylthio en $C_1$ à $C_4$; naphtyl-1 ou -2 ou un radical hétérocyclyle à 5 ou 6 chaînons et à un ou deux hétéroatomes choisis parmi le soufre, l'oxygène ou l'azote, éventuellement fusionné avec un cycle benzénique et le cas échéant substitué par un ou deux atomes d'halogène ou radicaux alkyle ou alkoxy en $C_1$ à $C_4$.

Suivant une forme préférée de l'invention, celle-ci a pour objet les composés de formule 1, dans laquelle $R_1$ et $R_2$ représentent indépendamment des atomes d'hydrogène ou d'halogène, des radicaux alkyle, phényle ou benzyle, $R_3$ et $R_4$ représentent des atomes d'hydrogène ou d'halogène, ou des radicaux alkyle ou alkoxy en $C_1$ à $C_6$, $R_5$ et $R_6$ représentent des atomes d'hydrogène ou des radicaux alkyle $C_1$ à $C_6$, ou alcènyle en $C_2$ à $C_6$ ou forment ensemble une chaîne alkylène en $C_4$, $R_7$ représente un atome d'hydrogène, $R_8$ représente un atome d'hydrogène ou un radical alkyle en $C_1$ à $C_8$ et $R_9$ désigne un groupe phényle non substitué ou portant un à trois substituants choisis parmi halogène et les radicaux alkyle et alkoxy en $C_1$ à $C_4$.

Par le terme "alkyle" on entend un enchaînement hydrocarboné, sauf précisions contraires, saturé, linéaire ou ramifié, dérivé de l'alcane correspondant par suppression d'un atome d'hydrogène et comprenant plus particulièrement 1 à 5 atomes de carbone comme par exemple : méthyle, éthyle, n-propyle, n-butyle, 2-méthyléthyle, 2,2-diméthyléthyle,ou 3,3-diméthylpropyle.

Par le terme "alcényle" on entend un enchaînement hydrocarboné, sauf précisions contraires, linéaire ou ramifié, comprenant plus précisement 3 à 6 atomes de carbone et contenant une double liaison, comme par exemple : prop-2-ényle, 2-méthylprop-2-ényle, but-3-ényle.

Par le terme "halogène" on entend plus précisement le brome, le chlore ou le fluor.

Les termes "alkoxy" ou "alkylthio" caractérisent un enchaînement alkyle tel que défini ci-dessus, lié à la molécule parent par l'intermédiaire d'un atome d'oxygène ou de soufre comme par exemple les radicaux : méthoxy, éthoxy, n.propoxy, 1-méthyléthoxy, n.butoxy, 1,1-diméthyléthoxy, méthylthio, éthylthio, n.propylthio ou 1-méthyléthylthio.

Par les termes "alkoxyalkyle" ou "alkylthioalkyle" on entend un enchaînement alkoxy ou alkylthio tel que défini ci-dessus, lié à la molécule parent par l'intermédiaire d'un radical alkyle tel que défini précédemment, linéaire, comme par exemple : méthoxyméthyle, méthoxyéthyle, 2-méthyléthoxyméthyle, méthylthiométhyle, éthylthiométhyle.

Le terme "cycloalkyle" caractérise un enchaînement hydrocarboné saturé, cyclique, dérivé d'un cyclane tel que le cyclopropane, le cyclopentane, le cyclohexane, le cycloheptane ou le cyclooctane, par suppression d'un atome d'hydrogène et le cas échéant substitué par un ou deux radicaux alkyles tels que définis précédemment, comme par exemple : 2-méthylcyclopentyle, 2-méthylcyclohexyle, 4,4-diméthylcyclohexyle.

Le terme "cycoalcényle" caractérise un radical cycloalkyle tel que défini ci-dessus et contenant une double liaison.

Par "radical hétérocyclyle à 5 ou 6 chaînons et à 1 ou 2 hétéroatomes", on entend un radical dérivé, par suppression d'un atome d'hydrogène, d'un cycle à 5 ou 6 chaînons de degré d'insaturation maximal et contenant un ou deux hétéroéléments choisis parmi l'oxygène, l'azote ou le soufre comme par exemple les cycles thiophène, furane, pyrrole, pyridine, thiazole, isothiazole, oxazole, isoxazole, imidazole, pyrimidine ou pyrazine, notamment les radicaux : thiényl- ou furyl-2 ou -3, pyrrolyl-1, oxazolyl-, thiazolyl-, ou imidazolyl-2, -4 ou -5, isoxazolyl- ou isothiazolyl-3, -4 ou -5, pyridyl-2, -3 ou -4, pyrimidyl-2 ou -5, ou pyrazinyl-2 ou -3; l'expression "éventuellement fusionné avec un cycle benzénique" caractérise les radicaux dérivés, par suppression d'un atome d'hydrogène, des bicycles résultant de la fusion des hétérocycles sus-nommés avec le cycle benzénique tels que les bicycles : benzothiophène, benzofurane, indole, benzimidazole, quinoléine, isoquinoléine, quinoxaline ou quinazoline, en particulier les radicaux benzo(b)thiényle, ou benzo(b)furyl-5 ou -6, indolyl-2, -3, -5 ou -6, benzimidazolyl-2, -5 ou -6, quinolyl-2, -3, -4, -5, -6 ou -7, isoquinolyl-1, -3, -4, -6 ou -7, quinoxalyl-2, -3, -6 ou -7 ou quinazolyl-2, -4, -6 ou -7.

Les composés de formule **1** peuvent renfermer un ou plusieurs centres d'asymétrie, notamment lorsque les substituants $R_5$, $R_6$ et $R_7$ sont différents, ce qui génère des diastéréoisomères, énantiomères et racémiques qui font également partie de l'invention.

Il entre dans la compétence normale du spécialiste d'isoler ou de synthétiser une forme optiquement active d'un composé de formule **1**, par exemple par dédoublement d'un racémique ou par synthèse au départ d'un composé optiquement actif et de déterminer les propriétés biologiques des isomères ainsi isolés suivant les expérimentations décrites ci-après.

Les mélanges de stéréoisomères peuvent être séparés par les méthodes usuelles à celui des stades de la synthèse qui est le plus approprié; par méthodes usuelles, on entend l'ensemble des procédés familiers au spécialiste comme par exemple la cristallisation, la chromatographie ou la formation de combinaisons avec des composés optiquement actifs.

Les composés de formule **1** peuvent exister sous une ou plusieurs formes tautomères qui font aussi partie de l'invention.

Comme composés spécifiques de l'invention on peut citer, à titre d'exemples uniquement, les composés suivants dont les formules développées figurent sur les dessins annexés :

**composé n°1** : $N_1$-[1-(7-azaindol-1-yl)cyclopentylméthyl]-$N_2$-(2,6-diisopropylphényl)urée;
**composé n°2** : $N_1$-[2-(7-azaindol-1-yl)-2-allylpent-4-ényl]-$N_2$-(2,6-diisopropylphényl)urée;
**composé n°3** : $N_1$-[2-(7-azaindol-1-yl)-2-allylpent-4-ényl]-$N_2$-(4-fluoro-2-méthylphényl)urée;
**composé n°4** : $N_1$-[2-(7-azaindol-1-yl)-2-phényléthyl]-$N_2$-(2,6-diisopropylphényl)urée;
**composé n°5** : $N_1$-[2-(7-azaindol-1-yl)-2-méthylpropyl]-$N_2$-(2,6-diisopropylphényl)urée;
**composé n°6** : $N_1$-[1-(1-méthyl-7-azaindol-3-yl)cyclopentylméthyl]-$N_2$-(2,6-diisopropylphenyl)urée;
**composé n°7** : $N_1$-[1-(5-azaindol-1-yl)cyclopentylméthyl]-$N_2$-(2,6-diisopropylphényl)urée;
**composé n°8** : $N_1$-[2-(7-azaindol-1-yl)hexyl]-$N_2$-(2,6-diisopropylphényl)urée;
**composé n°9** : $N_1$-[1-(3-éthyl-7-azaindol-1-yl)cyclopentylméthyl]-$N_2$-(2,6-diisopropylphényl)urée.
**composé n°10** : $N_1$-[1-(7-azaindol-1-yl)cyclohexylméthyl]-$N_2$-(2,6-diisopropylphényl)urée.
**composé n°11** : $N_1$-[1-(6-chloro-3-phényl-7-azaindol-1-yl)cyclopentylméthyl]-$N_2$-(2,6-diisopropylphényl)-urée.
**composé n°13** : $N_1$-[1-(3-éthyl-4-azaindol-1-yl)cyclopentylméthyl]-$N_2$-(2,6-diisopropylphényl)urée;
**composé n°14** : $N_1$-[4-(7-azaindol-1-yl)pyrane-4-ylméthyl]-$N_2$-(2,6-diisopropylphényl)urée;
**composé n°15** : $N_1$-[4-(7-azaindol-1-yl)pent-1-éne-5-yl]-$N_2$-(2,6-diisopropylphényl)urée;
**composé n°16** : $N_1$-[1-(2,3-diméthyl-7-azaindol-1-yl)cyclopentylméthyl]-$N_2$-(2,6-diisopropylphényl)urée;
**composé n°17** : $N_1$-[1-(3-benzyl-7-azaindol-1-yl)cyclopentylméthyl]-$N_2$-(2,6-diisopropylphényl)urée;
**composé n°18** : $N_1$-[1-(3-butyl-7-azaindol-1-yl)cyclopentylméthyl]-$N_2$-(2,6-diisopropylphényl)urée;
**composé n°19** : $N_1$-[1-(3-propyl-7-azaindol-1-yl)cyclopentylméthyl]-$N_2$-(2,6-diisopropylphényl)urée;
**composé n°20** : $N_1$-[1-(3-isopropyl-7-azaindol-1-yl)cyclopentylméthyl]-$N_2$-(2,6-diisopropylphényl)urée;
**composé n°21** : $N_1$-[1-(3-phényl-7-azaindol-1-yl)cyclopentylméthyl]-$N_2$-(2,6-diisopropylphényl)urée;
**composé n°22** : $N_1$-[2-(7-azaindol-1-yl)butyl]-$N_2$-(2,6-diisopropylphényl)urée;
**composé n°24** : $N_1$-[1-(3-méthylthio-7-azaindol-1-yl)cyclopentylméthyl]-$N_2$-(2,6-diisopropylphényl)urée;
**composé n°26** : $N_1$-[1-(5-chloro-3-éthyl-7-azaindol-1-yl)cyclopentylméthyl]-$N_2$-(2,6-diisopropylphényl)-urée;
**composé n°27** : $N_1$-[1-(6-azaindol-1-yl)cyclopentylméthyl]-$N_2$-(2,6-diisopropylphényl)urée;
**composé n°28** : $N_1$-[1-(4-méthyl-3-phényl-7-azaindol-1-yl)cyclopentylméthyl]-$N_2$-(2,6-diisopropylphényl)urée;
**composé n°29** : $N_1$-[1-(3-méthyl-7-azaindol-1-yl)cyclopentylméthyl]-$N_2$-(2,6-diisopropylphényl)urée;
**composé n°30** : $N_1$-[1-(3-(4-fluorophényl)-7-azaindol-1-yl)cyclopentylméthyl]-$N_2$-(2,6-diisopropylphényl)-urée;

**composé n°31** : $N_1$-[2-(3-phényl-7-azaindol-1-yl)hexyl]-$N_2$-(2,6-diisopropylphényl)urée;
**composé n°32** : $N_1$-[1-(3-(4-méthoxyphényl-7-azaindol-1-yl)cyclopentylméthyl]-$N_2$-(2,6-diisopropylphényl)urée;
**composé n°34** : $N_1$-(1-(3-hexyl-7-azaindol-1-yl)cyclopentylméthyl]-$N_2$-(2,6-diisopropylphényl)urée;
**composé n°35** : $N_1$-[1-(7-azaindol-1-yl)cyclopentylméthyl]-$N_2$-(2,4,6-triméthoxyphényl)urée;
**composé n°36** : $N_1$-[1-(7-azaindol-1-yl)cyclopentylméthyl]-$N_2$-(4,6-diméthoxypyrimidin-5-yl)urée;
**composé n°37** : $N_1$-[1-(7-azaindol-1-yl)cyclopentylméthyl]-$N_2$-(2,6-diéthylphényl)urée;
**composé n°38** : $N_1$-[1-(7-azaindol-1-yl)cyclopentylméthyl]-$N_2$-(2-isopropylphényl)urée;
**composé n°39** : $N_1$-[1-(7-azaindol-1-yl)cyclopentylméthyl]-$N_2$-(2,4-diisopropylphényl)urée;
**composé n°40** : $N_1$-[1-(7-azaindol-1-yl)cyclopentylméthyl]-$N_1$-benzyl-$N_2$-(2,4-difluorophényl)urée;
**composé n°41** : $N_1$-[1-(3-bromo-7-azaindol-1-yl)cyclopentylméthyl]-$N_2$-(2,6-diisopropylphényl)urée;
**composé n°42** : $N_1$-[1-(3-chloro-7-azaindol-1-yl)cyclopentylméthyl]-$N_2$-(2,6-diisopropylphényl)urée;
**composé n°45** : $N_1$-[1-(3-éthényl-7-azaindol-1-yl)cyclopentylméthyl]-$N_2$-(2,6-diisopropylphényl)urée;
**composé n°49** : chlorhydrate de $N_1$-[(7-azaindol-1-yl)cyclopentylméthyl]-$N_2$-(2,6-diisopropylphényl)urée;
**composé n°50** : $N_1$-[1-(7-azaindol-1-yl)cyclopentylméthyl]-$N_1$-benzyl-$N_2$-(2,6-diisopropylphényl)urée;
**composé n°51** : chlorhydrate de $N_1$-[2-(3-éthyl-7-azaindol-1-yl)hexyl]-$N_2$-(2,6-diisopropylphényl)urée.

L'invention concerne aussi les procédés de préparation des composés de formule **1**, caractérisés en ce qu'ils comportent au moins comme l'illustre le schéma 1 suivant,

a) la transformation d'un aminoazaindole de formule générale **5** dans laquelle, $X_1$, $X_2$, $X_3$, $X_4$, $R_1$, $R_2$, $R_3$, $R_4$, $R_5$, $R_6$ et $R_7$ ont les significations générales et particulières déja définies, en un aminoazaindole N-substitué de formule générale **4**; cette transformation peut être réalisée soit par alkylation par un composé halogéné de formule générale $R_8$-X dans laquelle, X désigne un atome de chlore, de brome ou d'iode et $R_8$ a les significations déja définies, soit par réaction avec un aldéhyde de formule $R_{10}$-CHO ou un chlorure ou un anhydride d'acide de formules générales $R_{10}$-COCl respectivement $(R_{10}\text{-CO})_2O$ dans lesquelles $R_{10}$ représente un radical alkyle en $C_1$ à $C_{11}$ ou phényle pouvant porter un à trois substituants choisis parmi halogène et les radicaux alkyle, alkoxy et alkylthio en $C_1$ à $C_4$, suivie de la réduction de l'imine ou de l'amide ainsi obtenus,

## Schéma 1

5 : $C(R_5)(R_6)\text{-}CH(R_7)\text{-}NH_2$ ; $X_1$, $X_2$, $X_3$, $X_4$, $R_1$, $R_2$, $R_3$, $R_4$

$R_8$-X/B(-) ou
$R_{10}$-CHO/réduction ou
$R_{10}$-COCl/réduction ou
$(R_{10}CO)_2O$/réduction

4 : $C(R_5)(R_6)\text{-}CH(R_7)\text{-}NH(R_8)$ ; $X_1$, $X_2$, $X_3$, $X_4$, $R_1$, $R_2$, $R_3$, $R_4$

$COCl_2/R_9NH_2$ ou
$R_9$-NCO ou
$Cl_3C\text{-}CO\text{-}NHR_9$ ou
$C_9H_5\text{-}O\text{-}CO\text{-}NHR_9$

1 : $C(R_5)(R_6)\text{-}CH(R_7)\text{-}N(R_8)\text{-}CO\text{-}NHR_9$ ; $X_1$, $X_2$, $X_3$, $X_4$, $R_1$, $R_2$, $R_3$, $R_4$

b) l'aminocarbonylation d'un aminoazaindole N-substitué obtenu selon a) et représenté par la formule générale **4** dans laquelle, $X_1$, $X_2$, $X_3$, $X_4$, $R_1$, $R_2$, $R_3$, $R_4$, $R_5$, $R_6$, $R_7$ et $R_8$ ont les significations générales

et particulières précédemment explicitées, par réaction avec le phosgène et une amine appropriée de formule générale $R_9$-$NH_2$ ou par réaction d'un isocyanate de formule générale $R_9$-NCO ou un trichloracétamide de formule générale $Cl_3C$-CO-$NHR_9$ ou bien encore un phénylcarbamate de formule générale $C_6H_5$-O-CO-$NHR_9$, dans lesquelles $R_9$ a les significations définies plus haut.

L'alkylation selon a) est réalisée en présence d'un agent basique, de préférence une amine tertiaire comme par exemple la triéthylamine, dans un solvant adapté, de préférence un solvant polaire comme par exemple le tétrahydrofurane et généralement à la température de reflux du solvant utilisé; la condensation d'un aldéhyde selon a) est effectuée dans un solvant ou un mélange de solvants inertes vis à vis des réactifs et non miscibles à l'eau; on peut utiliser comme solvants notamment des hydrocarbures aromatiques en particulier des alkylbenzènes comme le toluène ou le xylène; on peut le cas échéant, afin de faciliter la formation de l'imine, ajouter un agent de déshydratation comme par exemple l'acide paratoluènesulfonique; on opère de préférence à la température de reflux du solvant utilisé; l'imine ainsi obtenue est directement réduite en l'amine de formule générale **4** par le borohydrure de sodium; cette réduction est effectuée dans un solvant adapté comme le tétrahydrofurane ou un alcool qui peut être par exemple le méthanol ou bien l'éthanol et à une température comprise entre la température ambiante et la température de reflux du solvant utilisé; l'acylation selon a) est réalisée en présence d'un agent basique, de préférence une amine tertiaire aliphatique comme par exemple la triéthylamine, au sein d'un solvant qui est de préférence le tétrahydrofurane ou bien un hydrocarbure aromatique comme par exemple le benzène ou un alkylbenzène comme le toluène ou le xylène ou encore un solvant halogéné comme le chloroforme ou le chlorure de méthylène et de préférence à la température d'ébullition du solvant utilisé; l'amide ainsi obtenu est ensuite réduit par l'hydrure de lithium et d'aluminium dans l'éther éthylique ou le tétrahydrofurane, à la température d'ébullition du solvant utilisé.

La condensation d'un isocyanate selon b) est effectuée de préférence au sein d'un alcane comme le pentane ou l'hexane, ou encore d'un éther comme le diisopropyléther ou l'éther diéthylique ou bien, dans le cas d'une amine moins soluble, dans l'acétate d'éthyle et à la température la mieux adaptée pour obtenir la réaction qui est généralement la température ambiante; la réaction selon b), utilisant le phosgène et une amine de formule $R_9NH_2$, est réalisée au sein d'un hydrocarbure aromatique comme par exemple le toluène, en présence d'un agent basique comme la triéthylamine et à une température proche de la température ambiante; lorsque la formation du chlorure de carbamoyle intermédiaire est complète, on met à réagir ce dernier avec l'amine de formule $R_9NH_2$ souhaitée, à une température comprise entre la température ambiante et la température de reflux du solvant utilisé; l'aminocarbonylation selon b) dans laquelle on utilise un trichloracétamide de formule $Cl_3C$-CO-NH-$R_9$, est réalisée par chauffage au sein d'un solvant polaire aprotique comme par exemple le N,N-diméthylformamide, la tétraméthylurée, la N-méthylpyrrolidone ou encore l'hexaméthylphosphorotriamide, en présence d'un agent basique qui est de préférence un carbonate alcalin ou alcalino-terreux comme le carbonate de sodium ou de potassium et à une température comprise entre la température ambiante et 110°c, de préférence à une température proche de 110°c; l'aminocarbonylation selon b) dans laquelle on utilise un phénylcarbamate est réalisée dans un solvant polaire tel que le diméthylformamide à une température comprise entre la température ambiante et la température d'ébullition du solvant.

L'invention vise également les composés intermédiaires répondant aux formules générales **5, 6, 7** et **8** ainsi que leurs procédés de synthèse illustrés par le schéma **2** ci-après.

Les nitriles intermédiaires de formule générale **7** peuvent être préparés à partir des azaindoles appropriés de formule générale **9** dans laquelle $X_1$, $X_2$, $X_3$, $X_4$, $R_1$, $R_2$, $R_3$ et $R_4$ ont les significations générales ou particulières déja indiquées, selon le procédé décrit dans le brevet français 1.261.179 pour les composés ayant la chaîne acétonitrile en position -2 ou -3 ou selon le procédé décrit dans le brevet Belge 659467 pour les composés ayant la chaîne acétonitrile en position -1.

## Schéma 2

$R'_5$-CH=CH-$NO_2$ ; $R_5X/R_6X/B(-)$ ; $H_2$/cat ; réduction

$CH_2$-CN (7) → $C(R_5)(R_6)$-CN (6) ; 9 → $CH(R'_5)-CH_2-NO_2$ (8) ; → $C(R_5)(R_6)-CH(R_7)-NH_2$ (5)

Les nitriles de formule **7** peuvent être, le cas échéant, mono ou dialkylés par des halogénures de formule $R_5$-X ou $R_6$-X ou par un dihalogénure de formule X-$(CH_2)_q$-X, dans lesquelles, $R_5$ et $R_6$ ont les significations générales ou particulières spécifiées précédemment et le cas échéant renferment une double liaison et X désigne le chlore, le brome ou l'iode; cette réaction d'alkylation est réalisée en présence d'un agent basique comme l'hydrure de sodium ou encore un amidure alcalin ou alcalino-terreux comme l'amidure de sodium ou le diisopropylamidure de lithium, dans un solvant ou un mélange de solvants adaptés comme le diméthylformamide ou le benzène ou le toluène ou encore le diéthyléther et de préférence à une température généralement comprise entre 20 et 80°c, ou entre -50°c et la température ambiante dans le cas particulier où c'est un amidure de lithium qui est utilisé comme base.

Les nitriles (**6**) sont réduits en les amines (**5**); cette réduction consiste en une hydrogénation catalytique ou bien en une réduction chimique; l'hydrogénation catalytique est éffectuée à la pression atmosphérique ou sous une pression pouvant aller jusqu'à 180 bars, en présence d'un catalyseur métallique comme par exemple le palladium dispersé sur charbon ou le nickel de Raney et d'une base qui est généralement la soude en pastilles, l'ammoniac ou encore une amine tertiaire aliphatique comme la triéthylamine, au sein d'un solvant compatible avec l'hydrogène, de préférence polaire, comme par exemple le tétrahydrofurane ou un alcool comme le méthanol ou l'isopropanol; la réduction chimique est réalisée en utilisant comme réducteur de préférence l'hydrure de lithium et d'aluminium, bien que d'autres réducteurs de la fonction nitrile puissent également convenir, au sein d'un solvant usuellement utilisé avec ce réducteur comme le tétrahydrofurane ou le diéthyléther, à une température comprise entre la température ambiante et la température de reflux du solvant utilisé mais de préférence à sa température de reflux.

Un autre procédé pour obtenir les amines (**5**) consiste à condenser sur les azaindoles (**9**) un nitroalcène de formule générale $R'_5$-CH=CH-$NO_2$, dans laquelle $R'_5$ désigne un radical phényle pouvant porter des substituants choisis parmi halogène, alkyle, alkoxy ou alkylthio en $C_1$ à $C_4$; cette condensation qui est

effectuée à une température pouvant être comprise entre 50°c et 150°c conduit aux dérivés nitrés (**8**), lesquels sont ensuite réduits en les amines (**5**); cette réduction est de préférence effectuée catalytiquement, a la pression atmosphérique ou sous une pression pouvant aller jusqu'à 180 bars, en présence d'un catalyseur métallique comme par exemple le palladium dispersé sur charbon ou le nickel de Raney au sein d'un solvant compatible avec l'hydrogène, de préférence polaire, comme par exemple le tétrahydrofurane ou un alcool comme le méthanol, l'éthanol ou l'isopropanol, à une température comprise entre 50°c et sa température de reflux.

Les azaindoles de formule générale **9** sont préparés selon les techniques décrites dans Russian Chemical Reviews, 1980, 49(5), 428-444.

Les amines de formule **4** dans laquelle $R_7$ a les significations spécifiées précédemment autres que l'hydrogène, peuvent être obtenues selon le schéma **3** ci-après, selon lequel on prépare les cétones de formule générale **10** dans laquelle $X_1$, $X_2$, $X_3$, $X_4$, $R_1$, $R_2$, $R_3$, $R_4$, $R_5$, $R_6$ et $R_7$ ont les significations générales ou particulières déja indiquées, par action d'un magnésien sur les nitriles de formule générale **6** dans un solvant tel que l'éther diéthylique, le tétrahydrofurane ou dans un mélange de ces solvants comme le mélange benzène-éther, et à une température comprise entre 0°c et sa température d'ébullition; les cétones (**10**) ainsi obtenues sont transformées ensuite en les amines (**4**) par réaction avec une amine primaire de formule $R_8NH_2$ dans laquelle $R_8$ a les significations générales déja indiquées; cette réaction est effectuée dans un solvant ou un mélange de solvants, inertes vis à vis des réactifs et non miscibles à l'eau; à cet effet on peut utiliser notamment des hydrocarbures aromatiques, en particulier des alkylbenzènes comme le toluène ou le xylène et le cas échéant ajouter un agent de déshydratation comme par exemple l'acide paratoluènesulfonique; on opère de préférence à la température de reflux du solvant ou du mélange de solvants utilisés; les imines ainsi obtenues sont ensuite réduites *in situ* en les amines (**4**) correspondantes par le borohydrure de sodium; cette réduction est effectuée dans un solvant adapté comme le tétrahydrofurane ou un alcool qui peut être par exemple le méthanol ou bien l'éthanol et à une température comprise entre la température ambiante et la température de reflux du solvant utilisé.

## Schéma 3

$C(R_5)(R_6)$-CN (**6**) —$R_7$-MgX→ $C(R_5)(R_6)$-CO-$R_7$ (**10**) —1) $R_8$-$NH_2$ 2) réduction→ $C(R_5)(R_6)$-$CH(R_7)$-$NHR_8$ (**4**)

Les composés de formule 1 ont la propriété d'inhiber l'acyl coenzyme A:cholesterol acyl transférase (A.C.A.T.) et exercent une activité hypolipidémiante et anti-athéromateuse.

Ces propriétés des composés de l'invention rendent particulièrement intéressante leur utilisation comme médicament pour le traitement ou la prévention des hyperlipidémies et de l'athérosclérose.

Les propriétés pharmacologiques des composés de l'invention ont été démontrées par les tests suivants,

**Test A:** mesure de l'inhibition de l'A.C.A.T. aortique *in vitro* chez le lapin: des lapins néo-zélandais mâles pesant entre 2,2 et 2,5 kg, préalablement soumis pendant 15 jours à un régime enrichi de 1,25 %

en cholestérol, sont sacrifiés par dislocation cervicale; l'aorte est prélevée, disséquée et homogénéisée afin d'en préparer la fraction microsomiale par ultracentrifugation; ces microsomes sont incubés en présence de $^{14}$C-oléoyl coenzyme A conformément à la méthode décrite par P.J. GILLIES et coll., Exp. and Mol. Pathol., 1986, 44, 329-339; on extrait les lipides de l'incubat par un mélange méthanol-chloroforme et le $^{14}$C-oléoyl cholestérol est séparé par CCM: ce dernier représente la mesure de l'activité A.C.A.T. et les résultats sont exprimés sous forme de concentration inhibitrice 50 ($CI_{50}$) représentant la concentration de composé qui inhibe l'activité A.C.A.T. de 50 %.

**Test B:** mesure de l'effet hypocholestérolémiant chez le rongeur: des rats Wistar mâles de 200-220 g, sont soumis à un régime enrichi de 2,5 % en cholestérol pendant 8 jours; les deux derniers jours, ils sont traités oralement par le produit à tester, 24 heures et 4 heures avant leur sacrifice par exsanguination; la cholestérolémie est évaluée sur une fraction aliquote de sérum par une méthode enzymatique automatisée; les résultats sont exprimés sous forme de dose efficace 25 ($DE_{25}$) en mg par kg d'animal et qui représente la quantité de composé qui abaisse la cholestérolémie de 25 **%.**

**Test C:** mesure de l'inhibition de l'absorption intestinale du cholestérol chez le rat; des rats Wistar mâles de 230-250 g, à jeun depuis 24 heures, sont traités simultanément par le produit à tester administré *per os* et par du triton WR1339 administré par voie i.v.; une heure plus tard, on les traite à nouveau oralement par du $^{3}$H-cholestérol; trois heures plus tard, sous anesthésie à l'éther, on leur prélève 1 ml de sang au sinus rétro-orbitaire; la radioactivité sanguine, évaluée sur 0,1 ml de sérum, représente la mesure de l'absorption du $^{3}$H-cholestérol administré; les résultats sont exprimés sous forme de dose efficace 50 ($DE_{50}$) en mg par kg d'animal et qui. représente la quantité de composé qui inhibe l'absorption intestinale du cholestérol de 50 %.

On a par exemple obtenu pour le composé n°1 sur le test A, une $CI_{50}$ de $160x10^{-9}$ $Mole.l^{-1}$, sur le test B, une $DE_{25}$ de 0,202 $mg.kg^{-1}$, sur le test C, une $DE_{50}$ de 0,056 $mg.kg^{-1}$.

Les toxicités aigües des composés de l'invention ont été évaluées chez le rat et la souris; il s'est avéré, dans ces conditions, que les composés de l'invention sont particulièrement bien tolérés puisqu'à des doses supérieures à 3200 $mg.kg^{-1}$ *per os* il n'a été observé aucune mortalité.

Ces propriétés des composés de l'invention rendent particulièrement intéressante leur utilisation comme médicaments notamment pour le traitement des hyperlipidémies et de l'athérosclérose.

Les médicaments de l'invention sont caractérisés en ce qu'ils renferment une quantité efficace d'au moins un composé de formule générale **1** en association avec un véhicule pharmacologiquement acceptable et le cas échéant avec tout autre produit acceptable du point de vue pharmaceutique qui peut être inerte ou physiologiquement actif.

Ces médicaments peuvent être administrés selon une grande variété de formes d'administration différentes, par exemple sous forme solide, telles que comprimés, capsules, granules, gélules, poudres, suppositoires etc..., ou sous forme liquide telles que sirops, élixirs ou solutions injectables; dans ces compositions le principe actif peut être par exemple mélangé à un ou plusieurs diluants inertes tels que lactose, amidon, en outre ces compositions peuvent comprendre des substances autres que des diluants par exemple des lubrifiants tels que talc ou stéarate de magnésium; lorsqu'on désire des suspensions aqueuses, élixirs ou sirops pour administration orale, l'ingrédient actif essentiel peut y être associé à divers édulcorants et/ou aromatisants, le cas échéant des émulsionnants et/ou des agents de mise en suspension en même temps que des diluants tels que l'eau, l'éthanol, le propylèneglycol, et diverses associations similaires.

Ces compositions pharmaceutiques selon l'invention se présentent généralement sous forme de dose unitaire pouvant contenir une quantité de principe actif comprise entre 10 et 100 % de leur poids total et de préférence située dans l'intervalle 10 à 60 %.

Lorsque le composé de l'invention est utilisé comme agent hypolipidémiant ou anti-athéroscléreux, le dosage adopté et le nombre des administrations sont fonction du sexe, du poids et de l'acuité des symptômes du patient ainsi que de la nature et du degré de l'effet thérapeutique escompté. Généralement, par voie orale il est administré de préférence aux doses de 10 à 500 mg par jour en une ou plusieurs fractions ce qui correspond pour un adulte d'un poids moyen de 70 kg à un intervalle de doses d'environ 0,15 à 7 mg par kilo et par jour.

L'exemple suivant, donné à titre non limitatif, illustre une composition de ce type,

**Exemple:**

| | |
|---|---|
| Principe actif: composé n°1 | 50 mg |
| Lactose | 69 mg |
| Phosphate dicalcique | 69 mg |
| Carboxyméthyl cellulose sodique | 10 mg |
| Stéarate de magnésium | 2 mg |

L'invention est illustrée par les exemples non limitatifs décrits ci-après dans lesquels:

. toutes les évaporations sont exécutées, sauf indication contraire, dans un évaporateur rotatif sous pression réduite,

. les températures sont exprimées en degrés centigrade (°c),

. lorsqu'il est indiqué "température ambiante", il s'agit d'une température qui peut être comprise entre 18 et 25°c,

. sauf indication contraire, le degré d'avancement de la réaction est contrôlé par chromatographie en couche mince (CCM),

les nouveaux composés sont le cas échéant caractérisés par leurs constantes physiques: point de fusion noté F ou point d'ébullition noté Eb suivi éventuellement de l'indication de la pression en millibars,

. les spectres de résonnance magnétique nucléaire sont, sauf indication contraire, ceux du proton et sont relevés à 60 MHz en présence de tétraméthylsilane comme étalon inteme et les déplacements chimiques sont indiqués en parties par million (ppm); les signaux sont décrits par les abbréviations suivantes: s = singulet, d = doublet, dd = doublet de doublet, t = triplet, q = quartet, hept. = heptuplet, m = multiplet,

. les spectres infra-rouge des composés sont enregistrés à partir d'échantillons dispersés dans le bromure de potassium dans le cas des composés solides ou bien en film dans le cas des composés liquides.

**Exemple 1:**

**$N_1$-[(7-Azaindol-1-yl)cyclopentylméthyl]-$N_2$-(2,6-diisopropylphényl)urée (composé n°1, formule 1: $R_1=R_2=R_3=R_4=R_7=R_8=H$, $R_5$-$R_6$=-$(CH_2)_4$-, $R_9$=2,6-diisopropylphényl, $X_4$=N).**

**Stade 1:**

**1-(7-Azaindol-1-yl)cyclopentanenitrile.**

A une suspension de 1,4g de NaH dans 30 ml de DMF, on ajoute goutte à goutte une solution de 6,3g (0,029mole) de 1,4-dibromobutane et 4,16g de 7-azaindol-1-ylacétonitrile dans 25ml de DMF. On agite 4 heures à température ambiante puis on y ajoute ensuite 200 cm3 d'eau et on extrait au chlorure de méthylène. La phase organique est ensuite séchée sur sulfate de sodium puis filtrée; le filtrat est évaporé et le résidu obtenu est chromatographié sur 85g de gel de silice (éluant hexane -$CH_2Cl_2$ puis $CH_2Cl_2$). On obtient ainsi un liquide qui cristallise.
Rendement = 4,45g = 79%.
CCM ($SiO_2$/hexane-$CH_3CO_2C_2H_5$:3-1):Rf = 0,43.
IR: $\nu$CN = 2230cm$^{-1}$.
RMN ($CDCl_3$): 2,0(m,4H); 2,70(m,4H); 6,5(d,1H); 7,2(m,2H); 7,9(dd,1H); 8,4 (dd,1H).

**Stade 2:**

**1-(7-Azaindol-1-yl)cyclopentylméthylamine.**

On hydrogène pendant 6 heures à 70°c un mélange de 4,35g (0,0206 mole) du nitrile obtenu au stade 1 dans 60 ml d'éthanol préalablement saturé d'ammoniac et en présence de 2g de nickel de Raney. On filtre le mélange, lave le nickel par 140 ml d'alcool puis évapore la solution à sec. On isole ainsi un composé liquide.

Rendement = 3,88g = 87%.
IR: $\nu NH_2$ = 3380 $cm^{-1}$.
RMN ($CDCl_3$): 0,9(m,2H; 1,7(m,4H); 2,3(m,4H); 3,3(s,2H); 6,35-8,25(m,5H)

**Stade 3:**

**$N_1$-[(7-Azaindol-1-yl)cyclopentylméthyl]-$N_2$-(2,6-diisopropylphényl)urée (composé n°1).**

A une solution de 2,15g (0,01 mole) de composé obtenu au stade 2 dans 50 cm3 d'éther, on ajoute goutte à goutte à température ambiante, 2,03g (0,01 mole) de 2,6-diisopropylphénylisocyanate et on agite le mélange pendant une nuit à la température ambiante; on essore ensuite le précipité obtenu, on le lave au diisopropyléther puis a l'hexane et on le sèche. Poids = 2,15g F = 147-149 ° C. Le filtrat est évaporé à sec et chromatographié sur 46g de gel de silice (éluant: $CH_2Cl_2$-$CH_3CO_2C_2H_5$: 1-1). On obtient 1,5g de solide F = 143-145 ° C.
Rendement global = 90%
IR: $\nu CO$ = 1635 $cm^{-1}$.
RMN ($CDCl_3$): 0,95(s,6H); 1,05(s,6H);1,8(m,4H); 2,3(m,4H); 3,15(m,2H); 3,7(s,NH); 3,8(s,1H); 5,55(m,1H); 5,8-7,8(m,8H).

| Analyse centésimale ($C_{26}H_{34}N_4O$-PM = 418,59): | | | |
|---|---|---|---|
| | C | H | N |
| %calculé | 74,61 | 8,19 | 13,39 |
| %trouvé | 74,32 | 8,28 | 13,32 |

**Exemple 2:**

**$N_1$-[2-(7-Azaindol-1-yl)-2-allyl-4-pentényl)]-$N_2$-(2,6-diisopropylphényl)urée composé n°2, formule 1: $R_1$=$R_2$=$R_3$=$R_4$=$R_7$=$R_8$=H,$R_9$=2,6-diisopropyl phényle, $R_5$=$R_6$= $CH_2$=CH-$CH_2$-, $X_4$=N).**

**Stade 1:**

**2-(7-Azaindol-1-yl)-2-allylpent-4-ènenitrile.**

Préparé selon le mode opératoire du stade 1 de l'exemple 1, en utilisant deux équivalents de bromure d'allyle. Composé liquide.
Rendement = 70%.
RMN ($CDCl_3$): 3,05(dd,2H); 3,75(dd,2H); 4,8-6(m,6H); 6,4(d,1H); 7,04(dd,2H); 7,4(d,1H); 7,9(d,1H); 8,25-(d,2H).

**Stade 2:**

**2-(7-Azaindol-1-yl)-2-allylpent-4-èneamine.**

A une suspension de 0,96g (0,0253 mole) de $LiAlH_4$ dans 100ml d'éther, on ajoute 4g du composé du stade 1 dissous dans 40 ml d'éther. On chauffe à reflux 3 heures. On refroidit et ajoute de la soude 5% à température ambiante, on décante, on lave la phase aqueuse à l'éther, on sèche les phases éthérées sur sulfate de sodium, on filtre et on évapore le filtrat. On obtient ainsi un composé liquide.
Rendement = 3,3g = 82%
IR:$\nu NH_2$ = 3400 $cm^{-1}$.
RMN ($CDCl_3$): 1,1(d,1H); 2,6-3,4(m,4H); 3,5(s,2H); 4,75-5,9(m,6H); 6,4(d,1H); 7,0(dd,2H); 7,3(d,1H); 7,85-(d,1H); 8,25(d,1H).

**Stade 3:**

**$N_1$-[2-(7-Azaindol-1-yl)-2-allylpent-4-ényl)]-$N_2$-(2,6-diisopropylphényl)urée (composé n°2).**

Préparé à partir du composé du stade 2, par le procédé décrit au stade 3 de l'exemple 1.
Rendement = 70% F = 118-120 ° C.
IR $\nu$NH = 3207cm$^{-1}$; $\nu$CO = 1652 cm$^{-1}$.
RMN ($CDCl_3$): 1,0(d,12H); 2,8(d,4H); 3,25(m,2H); 4,0(d,2H); 5-5,5(m,6H); 6,0-8,0(m,10H).

| Analyse centésimale ($C_{28}H_{36}N_4O$-PM = 444,62): | | | |
|---|---|---|---|
| | C | H | N |
| %calculé:<br>%trouvé: | 75,64<br>75,65 | 8,16<br>8,26 | 12,60<br>12,36 |

**Exemple 3:**

**$N_1$-[2-(7-Azaindol-1-yl)-2-allylpent-4-ényl)]-$N_2$-(4-fluoro-2-méthylphényl)urée (composé n°3, formule 1: $R_1$=$R_2$=$R_3$=$R_4$=$R_7$=$R_8$=H,$R_9$=4-fluoro-2-méthylphényle, $R_5$=$R_6$=$CH_2$=CH-$CH_2$-, $X_4$=N).**

**Stade 1:**

**2,2,2-Trichloro-N-(4-fluoro-2-méthylphényl)acétamide.**

A une solution de 6,25g (0,05 mole) de 4-fluoro-2-méthylaniline dans 75 ml de dichlorométhane et 3,95g (0,05 mole) de pyridine on ajoute à -5 ° C une solution de 9,1g (0,05 mole) de chlorure de trichloroacétyle dans 40 ml de dichloroéthane. On agite cette solution à température ambiante pendant une heure, puis on la verse sur de l'eau glacée, on décante la phase organique, on la lave à l'eau et on la sèche sur sulfate de sodium. On filtre, on évapore le filtrat et on disperse le résidu dans l'hexane, on obtient ainsi un solide de couleur rose.
Rendement = 10,25g = 75%. F = 119-121 ° C.
IR $\nu$CO = 1698 cm$^{-1}$.

**Stade 3:**

**$N_1$-[2-(7-Azaindol-1-yl)-2-allylpent-4-ényl)]-$N_2$-(4-fluoro-2-méthylphényl)urée (composé n° 3)**

On chauffe une heure à 110 ° C un mélange de 1,2g (1,45 mmole) du composé obtenu au stade 1, 1,58g de $K_2CO_3$ et 1,13g (1,45 mmole) d'amine obtenue au stade 2 de l'exemple 2 dans 15 ml de DMF. On verse le mélange réactionnel sur de l'eau glacée puis on extrait à l'ether, on lave à l'eau la phase organique et on la sèche sur sulfate de sodium. Après filtration puis évaporation du filtrat on obtient 1,4g d'un solide que l'on chomatographie sur silice (éluant hexane-$CH_2Cl_2$ puis $CH_2Cl_2$-$CH_3CO_2C_2H_5$:5-1). Rendement = 0,45g = 25%
F = 150-152 ° C(diisopropyléther)
IR $\nu$NH = 3344 cm$^{-1}$; $\nu$CO = 1632 cm$^{-1}$.
RMN ($CDCl_3$): 2,1(s,3H); 2,9(m,4H); 4(d,2H); 4,8-6(m,7H); 6,2(s,1H); 6,3-8,0(m,8H).

| Analyse centésimale ($C_{23}H_{25}N_4OF$-PM = 392,48) : | | | | |
|---|---|---|---|---|
| | C | H | N | F |
| %calculé:<br>%trouvé: | 70,39<br>70,27 | 6,42<br>6,61 | 14,28<br>14,33 | 4,84<br>4,86 |

**Exemple 4:**

**$N_1$-[2-(7-Azaindol-1-yl)-2-phényléthyl)]-$N_2$-(2,6-diisopropylphényl)urée (composé n°4, formule 1: $R_1=R_2=R_3=R_4=R_5=R_7=R_8=H$, $R_9$=2,6-diisopropylphényle, $R_6$=phényle, $X_4$=N).**

**Stade 1:**

**1-(2-Nitro-1-phényléthyl)-7-azaindole.**

On chauffe à 80°C un mélange de 2,36g (0,02 mole) de 7-azaindole et 3,3g de 2-phényl-1-nitroéthylène pendant 5 heures. On chromatographie le mélange réactionnel sur 112g de silice (éluant: hexane-$CH_2Cl_2$:1-1 puis $CH_2Cl_2$ puis $CH_2Cl_2$-$CH_3CO_2C_2H_5$:5-1).
Rendement = 2,4g = 44%
RMN ($CDCl_3$): 5,15(dd,1H); 5,7(dd,1H); 6,4-7,5(m,8H); 7,85(d,1H); 8,3(d,1H).

**Stade 2:**

**2-(7-Azaindol-1-yl)-2-phényléthylamine.**

On hydrogène sous pression à 70°C 2,15g (0,008 mole) du composé obtenu au stade 1 dans 50 ml d'éthanol en présence de 1g de nickel de Raney. On filtre le mélange réactionnel et on évapore le filtrat à sec. On obtient un composé liquide.
Rendement = 1,8g = 94%.
IR: $\nu NH_2$ = 3367 $cm^{-1}$.
RMN ($CDCl_3$): 2,35(s,2H); 3,6(d,2H); 6-8,4(m,11H).

**Stade 3:**

**$N_1$-[2-(7-Azaindol-1-yl)-2-phényléthyl)]-$N_2$-(2,6-diisopropylphényl)urée (composé n°4).**

Préparé à partir du composé du stade 2, par le procédé décrit au stade 3 de l'exemple 1.
Rendement = 65% F = 171-173°C.
CCM ($SiO_2$/hexane-$CH_3CO_2C_2H_5$:1-1): $R_f$ = 0,6.
RMN ($CDCl_3$): 0,95(d,12H); 3,0(m,2H); 4,1(m,2H); 5,1(m,1H); 5,9(m,2H); 6,3-8,0(m,13H).

| Analyse centésimale ($C_{28}H_{32}N_4O$-PM = 440,50): | | | |
|---|---|---|---|
| | C | H | N |
| %calculé: | 76,33 | 7,32 | 12,72 |
| %trouvé: | 76,54 | 7,36 | 12,84 |

**Exemple 5:**

En utilisant les procédés appropriés des exemples 1 à 4, on a préparé les composés (voir tableau 1 ci-dessous) de formule générale **1** dans laquelle $R_4 = R_7 = R_8 = H$ et $R_9$ = 2,6-diisopropylphényle.
Les abréviations Me, Et, nBu et Ph utilisées dans le tableau ci-dessous, désignent respectivement les radicaux : $CH_3$-, $CH_3CH_2$-, $CH_3$-$(CH_2)_2$-$CH_2$- et $C_6H_5$-; la notation $iC_3H_7$ utilisée sur les dessins annexés désigne le radical: $(CH_3)_2CH$-.

TABLEAU 1

| composé n° | $X_1$ à $X_4$ | R1 | R2 | $R_3$ | R5 | R6 | F°C |
|---|---|---|---|---|---|---|---|
| **5** | $X_4$=N | 2-H | 3-H | H | Me | Me | 140-142 |
| **6** | $X_4$=N | 1-Me | 2-H | H | -$(CH_2)_4$- | | 186-188 |
| **7** | $X_2$=N | 2-H | 3-H | H | -$(CH_2)_4$- | | 195,5-198 |
| **8** | $X_4$=N | 2-H | 3-H | H | n.Bu | H | 153-155 |
| **9** | $X_4$=N | 2-H | 3-Et | H | -$(CH_2)_4$- | | 142-144 |
| **10** | $X_4$=N | 2-H | 3-H | H | -$(CH_2)_5$- | | 133-135 |

| 11 | $X_4$=N | 2-H | 3-Ph | 6-Cl | -$(CH_2)_4$- | | amorphe |
|---|---|---|---|---|---|---|---|
| 13 | $X_1$=N | 2-H | 3-Et | H | -$(CH_2)_4$- | | 194-196 |
| 14 | $X_4$=N | 2-H | 3-H | H | -$(CH_2)$-O-$(CH_2)_2$- | | 152-154 |
| 15 | $X_4$=N | 2-H | 3-H | H | allyl | H | 153-155 |
| 16 | $X_4$=N | 2-Me | 3-Me | H | -$(CH_2)_4$- | | 157-159 |
| 17 | $X_4$=N | 2-H | 3-$CH_2$-Ph | H | -$(CH_2)_4$- | | 129-131 |
| 18 | $X_4$=N | 2-H | 3-n.Bu | H | -$(CH_2)_4$- | | 101-103 |
| 19 | $X_4$=N | 2-H | 3-n.propyl | H | -$(CH_2)_4$- | | 113-115 |
| 20 | $X_4$=N | 2-H | 3-isopropyl | H | -$(CH_2)_4$- | | 134-136 |
| 21 | $X_4$=N | 2-H | 3-Ph | H | -$(CH_2)_4$- | | 75-78 |
| 22 | $X_4$=N | 2-H | 3-H | H | Et | H | 180-182 |
| 24 | $X_4$=N | 2-H | 3-$SCH_3$ | H | -$(CH_2)_4$- | | 119-121 |
| 26 | $X_4$=N | 2-H | 3-Et | 5-Cl | -$(CH_2)_4$- | | 141-142 |
| 27 | $X_3$=N | 2-H | 3-H | H | -$(CH_2)_4$- | | 184-186 |
| 28 | $X_4$=N | 2-H | 3-Ph | 4-Me | -$(CH_2)_4$- | | 164-166 |
| 29 | $X_4$=N | 2-H | 3-Me | H | -$(CH_2)_4$- | | 132-134 |
| 30 | $X_4$=N | 2-H | 3-(4-fluoro phényl) | H | -$(CH_2)_4$- | | amorphe |
| 31 | $X_4$=N | 2-H | 3-Ph | H | n.Bu | H | 152-154 |
| 32 | $X_4$=N | 2-H | 3-(4-méthoxyphényl) | H | -$(CH_2)_4$- | | 150-151 |
| 34 | $X_4$=N | 2-H | 3-n.hexyl | H | -$(CH_2)_4$- | | liquide |

**Exemple 6:**

**$N_1$-[(7-Azaindol-1-yl)cyclopentylméthyl]-$N_2$-(2,4,6-triméthoxyphényl)urée (composé n°35 , formule 1: $R_1$=$R_2$=$R_3$=$R_4$=$R_7$=$R_8$=H', $R_5$-$R_6$=-$(CH_2)_4$-, $R_9$=2,4,6-triméthoxyphényl, $X_4$=N).**

Préparé à partir du composé du stade 2 de l'exemple 1, par le procédé décrit au stade 2 de l'exemple 3 en utilisant le 2,2,2-trichloro-N-(2,4,6-triméthoxyphényl)acétamide.
Rendement = 62% F = 163-164 °C
IR: $\nu$NH = 3394 $cm^{-1}$; $\nu$CO = 1669 $cm^{-1}$.
RMN ($CDCl_3$): 1,5- 2,8(m,8H); 3,6(s,6H); 3,75(s,3H); 4,5-5,8(m,3H); 6(s,2H); 6,25(s, 1H); 6,5-7,5(m, 2H); 7,5-8,1(m, 2H).

| Analyse centésimale ($C_{23}H_{28}N_4O_4$-PM = 424,48): | | | |
|---|---|---|---|
| | C | H | N |
| %calculé:<br>%trouvé: | 65,08<br>65,30 | 6,65<br>6,61 | 13,20<br>13,33 |

**Exemple 7:**

En utilisant le procédé de l'exemple 6, on a préparé les composés (voir tableau 2 ci-dessous) de formule générale **1** dans laquelle $R_1 = R_2 = R_3 = R_4 = R_7 = R_8 = H$, $R_5$-$R_6$ = -$(CH_2)_4$- et $X_4$ = N.

TABLEAU 2

| composé n° | $R_9$ | F°C |
|---|---|---|
| **36** | 4,6-diméthoxypyrimidin-5-yl | 174-176 |
| **37** | 2,6-diéthylphényl | 182-184 |
| **38** | 2-isopropylphényl | 184-186 |
| **39** | 2,4-diisopropylphényl | 174-176 |

**Exemple 8:**

**$N_1$-[(7-Azaindol-1-yl)cyclopentylméthyl]-$N_1$-benzyl-$N_2$-(2,4-difluorophényl)urée(composé n°40 , formule 1: $R_1$=$R_2$=$R_3$=$R_4$=$R_7$=H, $R_8$=benzyle, $R_9$= 2,4-difluorophényl), $R_5$ , $R_6$ = -$(CH_2)_4$-, $X_4$ = N.**

**Stade 1:**

**N-Benzylidène-N-1-(7-azaindol-1-yl)cyclopentylméthylamine.**

On chauffe 1h30 à reflux en éliminant l'eau formée, un mélange de 7g du composé préparé au stade 2 de l'exemple 1, 100ml de toluène et 3,8g de benzaldéhyde. On évapore à sec sous 1 mm/Hg et obtient 9,75g d'un composé liquide.
IR: $\nu$CN = 1645 $cm^{-1}$. Rendement = 98%.

**Stade 2:**

**N-Benzyl-N-1-(7-azaindol-1-yl)cyclopentylméthylamine.**

A une solution de 9,75g de composé du stade 1 dans 100ml de méthanol on ajoute par petite portions 1,21g de borohydrure de sodium en maintenant la température entre 0 et 5°C. On agite 10 mn à 0°C puis 2 heures à température ambiante. On verse dans l'eau glacée, on extrait à l'éther, on lave l'extrait éthéré avec une solution de saumure et on le sèche sur $Na_2SO_4$. On évapore le solvant.
IR: $\nu$NH = 3340 $cm^{-1}$. Rendement = 8,4g = 85%.
RMN ($CDCl_3$): 1- 3,6(m,9H); 3,1(s,2H); 3.45(s,2H); 6,3(d,1H); 6,7-7,5(m, 7H); 7,8(dd, 1H); 8,2(dd, 1H).

**Stade 3:**

**$N_1$-[(7⁻Azaindol-1-yl)cyclopentylméthyl]-$N_1$-benzyl-$N_2$-(2,4-difluorophényl)urée :**

A un mélange de 3,97g du composé du stade 2 dans 50ml d'hexane on ajoute goutte à goutte à une température inférieure à 20°C 1,8g de 2,4-difluorophénylisocyanate. On agite le mélange pendant une nuit puis on essore le précipité formé, on le lave à l'hexane et on le recristallise dans 120ml de diisopropyléther.
IR: $\nu$NH = 3306 $cm^{-1}$; $\nu$CO = 1638 $cm^{-1}$. Rendement = 3,3g = 55%.

CCM ($SiO_2$/hexane-AcOEt:1-1): Rf = 0,66.
RMN ($CDCl_3$): 1,5- 2,8(m,8H); 4,05(s,2H); 4,1(s,2H); 6,3-8,2(m, 14H).

| Analyse centésimale ($C_{27}H_{26}F_2N_4O$-PM = 460,53): | | | | |
|---|---|---|---|---|
| | C | H | N | F |
| %calculé:<br>%trouvé: | 70,43<br>70,66 | 5,69<br>5,77 | 12,17<br>12,16 | 8,25<br>8,20 |

**Exemple 9:**

**$N_1$-[(3-Bromo-7-azaindol-1-yl)cyclopentylméthyl]-$N_2$-(2,6-diisopropylphényl)urée (composé n°41, formule 1: $R_1$=$R_3$=$R_4$=$R_7$=$R_8$=H, $R_2$=Br, $R_5$-$R_6$=-$(CH_2)_4$-, $R_9$=2,6-diisopropylphényl, $X_4$=N).**

A une solution de 2g du composé n°1 dans 20ml de choroforme on ajoute goutte à goutte une solution de 0,76g de brome dans 9ml de tétrachlorure de carbone; cette addition est réalisée à une température comprise entre 0 et 10°C. On agite ensuite le mélange pendant 2heures à température ambiante puis on le neutralise à la soude, on extrait au chorure de méthylène, on lave à l'eau l'extrait organique, on le sèche sur $Na_2SO_4$, on filtre et on évapore le filtrat à sec. On recristallise le résidu obtenu dans 60ml de diisopropyléther.
Rendement = 1,1g = 46%. F = 137-139°C.
CCM ($SiO_2$/hexane-AcOEt 1-1): Rf = 0,69.
RMN ($CDCl_3$): 1 (d,12H); 1,8(m,4H); 2,2(m,4H); 3,1(m, 2H); 3,7(d, 2H); 5,3(m, 1H); 5,9(s, 1H); 6,75-7,9-(m,14H).

| Analyse centésimale ($C_{26}H_{33}BrN_4O$-PM = 497,48): | | | | |
|---|---|---|---|---|
| | C | H | N | Br |
| %calculé:<br>%trouvé: | 62,77<br>62,78 | 6,69<br>6,81 | 11,26<br>11,35 | 16,06<br>16,16 |

**Exemple 10:**

**$N_1$-[(3-Chloro-7-azaindol-1-yl)cyclopentylméthyl]-$N_2$-(2,6-diisopropylphényl)urée (composé n°42, formule 1: $R_1$=$R_3$=$R_4$=$R_7$=$R_8$=H, $R_2$=Cl, $R_5R_6$=-$(CH_2)_4$-, $R_9$=2,6-diisopropylphényl, $X_4$=N).**

A une solution de 2,64g du composé 1 dans 75 ml de méthanol on ajoute par petites portions 0,93g de N-chlorosuccinimide en maintenant la température du mélange réactionnel en dessous de 25°C. On agite ensuite pendant 5 heures à température ambiante puis on rajoute 0,186g de N-chlorosuccinimide et laisse sous agitation pendant une nuit. On reprend le mélange par l'eau, on extrait à l'ether, on lave à l'eau, l'extrait éthéré, et on le sèche sur $Na_2SO_4$. Le produit brut est purifié par chromatographie sur silice (éluant hexane-acétate-d'éthyle:3-1 puis 2-1); le produit obtenu est recristallisé dans l'heptane.
Rendement = 0,8g = 28%. F = 153-155°C.
CCM ($SiO_2$/hexane-AcOEt:1-1): Rf = 0,65.
RMN ($CDCl_3$): 1(d,12H); 1,8(m,4H); 2,2(m,4H); 3,1(m, 2H); 3,75(d, 2H); 5,3(m, 1H); 6,0(s, 1H); 6,8-7,9-(m,14H).

| Analyse centésimale ($C_{26}H_{33}ClN_4O$-PM = 453,03): | | | | |
|---|---|---|---|---|
| | C | H | N | Cl |
| %calculé:<br>%trouvé: | 68,93<br>68,72 | 7,34<br>7,75 | 12,37<br>12,30 | 7,83<br>8,13 |

**Exemple 13:**

**$N_1$-[(3-Ethényl-7-azaindol-1-yl)cyclopentylméthyl]-$N_2$-(2,6-diisopropylphényl)urée (composé n°45, formule 1: $R_1$=$R_3$=$R_4$=$R_7$=$R_8$=H, $R_2$=3-CH=$CH_2$, $R_5$-$R_6$=-$(CH_2)_4$-, $R_9$=2,6-diisopropylphényl, $X_4$=N).**

**Stade 1:**

**1-(3-Ethényl-7-azaindol-1-yl)cyclopentanenitrile.**

A une solution de 3,6g de bromure de méthyltriphénylphosphonium dans 50ml de THF, on ajoute goutte à goutte 6,25 ml d'une solution 1,6 N de n-butyl lithium dans l'hexane, on agite le mélange pendant 15 min à température ambiante puis y ajoute 2,4g du composé préparé au stade 1 de l'exemple 11 en solution dans 25ml de THF. On agite le mélange pendant 24heures puis on le chauffe à reflux pendant 4h30. On le dilue à l'eau puis on l'extrait au chorure de méthylène, on séche la phase organique sur sulfate de sodium, on filtre, on évapore le filtrat et on le chromatographie sur silice (éluant $CH_2Cl_2$).
Rendement = 1,4g = 58%.
CCM ($SiO_2$/$CH_3CO_2C_2H_5$): Rf = 0,94.

**Stade 2:**

**1-(3-Ethényl-7-azaindol-1-yl)cyclopentylméthylamine.**

Préparé à partir du composé du stade 1 ci-dessus par le procédé décrit au stade 2 de l'exemple 2.
Rendement = 99%. Le produit est utilisé brut dans l'étape suivante.

**Stade 3:**

**$N_1$-[(3-Ethényl-7-azaindol-1-yl)cyclopentylméthyl]-$N_2$-(2,6-diisopropylphényl)urée (composé n°45).**

Préparé à partir du composé du stade 2 par le procédé décrit au stade 3 de l'exemple 1. Le composé obtenu est chromatographié sur gel de silice (éluant AcOEt-hexane :7-3 puis 1-1).
CCM ($SiO_2$/ AcOEt-hexane:8-2): Rf = 0,85.
Rendement = 70%. Solide amorphe.
IR: $\nu$CO = 1669cm$^{-1}$.

| Analyse centésimale ($C_{28}H_{36}N_4O$-PM = 444,6): | | | |
|---|---|---|---|
| | C | H | N |
| %calculé: | 75,64 | 8,16 | 12,60 |
| %trouvé: | 75,61 | 8,24 | 12,88 |

**Exemple 17:**

**Chlorhydrate de $N_1$-[(7-azaindol-1-yl)cyclopentylméthyl]-$N_2$-(2,6-diisopropylphényl)urée (composé n°49, formule 1: $R_1$=$R_2$=$R_3$=$R_4$=$R_7$=$R_8$=H, $R_5$-$R_6$=-$(CH_2)_4$-, $R_9$=2,6-diisopropylphényl, $X_4$=N).**

On dissous 1,05g du composé 1 dans 25 ml d'éthanol et on ajoute 1 ml d'une solution 6N d'HCl dans l'éthanol. Après 48heures d'agitation on évapore, on reprend par 25 ml d'acétate déthyle, on chauffe à reflux, on refroidit et filtre.
Rendement = 0,8g = 72%. F = 178-180 ° C.
IR: $\nu$NH = 3222, 3263 et 3333 cm$^{-1}$; $\nu$CO = 1685 cm$^{-1}$.
RMN ($CDCl_3$): 1,05(d, 12H); 1,8-2,45(m,8H); 3(m, 2H); 3,9(s, 2H); 6-8(m, 8H).

| Analyse centésimale ($C_{26}H_{35}ClN_4O$-PM = 455,03): | | | | |
|---|---|---|---|---|
| | C | H | N | Cl |
| %calculé: | 68,63 | 7,75 | 12,31 | 7,79 |
| %trouvé: | 68,97 | 7,88 | 12,12 | 7,86 |

**Exemple 18:**

**$N_1$-[(7-Azaindol-1-yl)cyclopentylmèthyl]-$N_1$-benzyl-$N_2$-(2,6-diisopropylphényl)urée (composé n°50 , formule 1: $R_1$=$R_2$=$R_3$=$R_4$=$R_7$=H, $R_5$-$R_6$=-$(CH_2)_4$-, $R_8$=benzyl, $R_9$= 2,6-diisopropylphényl, $X_4$=N).**

Préparé à partir du composé préparé au stade 2 de l'exemple 8 par le procédé décrit au stade 3 de l'exemple 1. Le composé obtenu est chromatographié sur gel de silice (éluant $CH_2Cl_2$-hexane:1-1).
Rendement = 53%. F = 70-72 ° C.
IR: $\nu$CO = 1651cm$^{-1}$.
RMN ($CDCl_3$): 1,1(d, 12H); 1,8(m,4H); 2,5(m, 4H); 3,0(m, 2H); 4,0(s, 3H); 4,1(s, 2H); 6,4-8,1(m,13H).

| Analyse centésimale ($C_{33}H_{40}N_4O$-PM = 508,71): | | | |
|---|---|---|---|
| | C | H | N |
| %calculé: | 77,92 | 7,93 | 11,01 |
| %trouvé: | 78,18 | 8,05 | 11,16 |

**Exemple 19:**

**Chlorhydrate de $N_1$-[2-(3-éthyl-7-azaindol-1-yl)hexyl]-$N_2$-(2,6-diisopropyl phényl)urée (composé n°51 , formule 1: $R_1$=Et, $R_2$=$R_3$=$R_4$=$R_6$=$R_7$=$R_8$=H, $R_5$=Bu, $R_9$= 2,6-diisopropylphényl).**

La base correspondante est préparée à partir du 2-(3-éthyl-7-azaindol-1-yl)hexanamine par le procédé décrit au stade 3 de l'exemple 1. On en prépare le chlorhydrate selon le procédé de l'exemple 17.
Rendement = 46%. F = 130-133 ° C.
IR: $\nu$CO = 1680cm$^{-1}$.

| Analyse centésimale ($C_{28}H_{41}NCl_4O$-PM = 485,12): | | | | |
|---|---|---|---|---|
| | C | H | N | Cl |
| %calculé: | 69,33 | 8,52 | 11,55 | 7,31 |
| %trouvé: | 69,12 | 8,62 | 11,42 | 7,27 |

## Revendications

1. Composés de formule 1

$$
\text{Structure: azaindole ring with } X_1 \text{ (4)}, X_2 \text{ (5)}, X_3 \text{ (6)}, X_4 \text{ (7)}, N \text{ (1)}, \text{ positions 2, 3; substituents } R_1, R_2, R_3, R_4 \text{ and } C(R_5)(R_6)\text{-}CH(R_7)\text{-}N(R_8)\text{-}CO\text{-}NH\text{-}R_9
$$

**1**

dans laquelle

l'un des groupes $X_1$ à $X_4$ désigne un atome d'azote et les autres représentent un radical CH;

$R_1$ et $R_2$, qui peuvent être situés en position -1, -2 ou -3 du cycle azaindole, représentent chacun un atome d'hydrogène, un radical alkyle linéaire ou ramifié en $C_1$ à $C_{12}$, alcényle en $C_2$ à $C_6$, cycloalkyle ou cycloalcényle en $C_3$ à $C_8$, alkylthio en $C_1$ à $C_6$ ou lorsqu'ils sont en position -2 ou -3 du azaindole, un atome d'halogène; phényle ou benzyle non substitués ou portant un à trois substituants choisis parmi halogène et les radicaux alkyle, alkoxy et alkylthio en $C_1$ à $C_4$;

$R_3$ et $R_4$ qui peuvent être situés sur les sommets -4, -5, -6 ou -7 du cycle azaindole à condition que ceux-ci figurent un atome de carbone, représentent chacun un atome d'hydrogène ou d'halogène, alkyle, alkoxy ou alkylthio en $C_1$ à $C_6$ ou cycloalkyle en $C_3$ à $C_8$;

le groupement $-C(R_5)(R_6)-CH(R_7)-N(R_8)-CO-NH-R_9$ peut être lié aux sommets -1, -2 ou -3 du cycle azaindole, étant entendu que lorsque l'atome d'azote en position -1 du cycle azaindole n'est pas substitué par l'un des groupes $R_1$, $R_2$ ou $-C(R_5)(R_6)-CH(R_7)-N(R_8)CONHR_9$, il porte un atome d'hydrogène;

$R_5$ et $R_6$ représentent chacun un atome d'hydrogène, un radical alkyle en $C_1$ à $C_{12}$, alcényle en $C_2$ à $C_6$, cycloalkyle ou cycloalcényle en $C_3$ à $C_8$, alkoxyalkyle ou alkylthioalkyle en $C_2$ à $C_{12}$, ou bien

$R_5$ et $R_6$ forment ensemble une chaîne alkylène de formule $-(CH_2)_p-$ éventuellement substitué par un ou deux radicaux alkyle en $C_1$ à $C_4$ et dans laquelle p peut prendre les valeurs 3 à 7, ou une chaîne alkyloxyalkylène de formule $-(CH_2)_x-O-(CH_2)-_y$, dans laquelle x et y peuvent chacun prendre les valeurs 1 ou 2;

$R_7$ désigne un atome d'hydrogène, un radical alkyle en $C_1$ à $C_6$ ou cycloalkyle en $C_3$ à $C_8$;

l'un des substituants $R_5$ à $R_7$ peut aussi représenter un radical phényle ou benzyle non substitués ou portant un à trois substituants choisis parmi halogène et les radicaux alkyle, alkoxy et alkylthio en $C_1$ à $C_4$;

$R_8$ désigne un atome d'hydrogène, un radical alkyle linéaire ou ramifié en $C_1$ à $C_{12}$, un radical cycloalkyle en $C_3$ à $C_8$ ou un radical benzyle non substitué ou portant 1 à 3 substituants choisis parmi halogène et les radicaux alkyle, alkoxy ou alkylthio en $C_1$ à $C_4$;

$R_9$ désigne un radical phényle non substitué ou portant 1 à 3 substituants choisis parmi halogène et les radicaux alkyle, alkoxy et alkylthio en $C_1$ à $C_4$; naphtyl-1 ou -2 ou un radical hétérocyclyle à 5 ou 6 chaînons et à un ou deux hétéroatomes choisis parmi le soufre, l'oxygène ou l'azote, éventuellement fusionné avec un cycle benzénique et le cas échéant substitué par un ou deux atomes d'halogène ou radicaux alkyle ou alkoxy en $C_1$ à $C_4$.

2. Composés selon la revendication 1, répondant à la formule 1 dans laquelle,

$R_1$ et $R_2$ représentent indépendamment des atomes d'hydrogène ou d'halogène, des radicaux alkyle, phényle ou benzyle, $R_3$ et $R_4$ représentent des atomes d'hydrogène ou d'halogène, ou des radicaux alkyle ou alkoxy en $C_1$ à $C_6$, $R_5$ et $R_6$ représentent des atomes d'hydrogène des radicaux alkyle en $C_1$ à $C_6$, alcényle en $C_2$ à $C_6$ ou forment ensemble une chaîne alkylène en $C_4$, $R_7$ représente un atome d'hydrogène, $R_8$ représente un atome d'hydrogène ou un radical alkyle en $C_1$ à $C_8$ et $R_9$

désigne un groupe phényle non substitué ou portant un à trois substituants choisis parmi halogène et les radicaux alkyle et alkoxy en $C_1$ à $C_4$.

**3.** Composés choisis dans le groupe comprenant :

- la $N_1$-[1-(7-azaindol-1-yl)cyclopentylméthyl]-$N_2$-(2,6-diisopropylphényl)urée;
- la $N_1$-[2-(7-azaindol-1-yl)-2-allylpent-4-ényl)]-$N_2$-(2,6-diisopropylphényl)urée;
- la $N_1$-[2-(7-azaindol-1-yl)-2-allylpent-4-ényl)]-$N_2$-(4-fluoro-2-méthylphényl)urée;
- la $N_1$-[2-(7-azaindol-1-yl)-2-phényléthyl)]-$N_2$-(2,6-diisopropylphényl)urée;
- la $N_1$-[2-(7-azaindol-1-yl)-2-méthylpropyl)]-$N_2$-(2,6-diisopropylphényl)urée;
- la $N_1$-[1-méthyl-7-azaindol-3-yl)cyclopentylméthyl]-$N_2$-(2,6-diisopropylphényl)urée;
- la $N_1$-[1-(5-azaindol-1-yl)cyclopentylméthyl]-$N_2$-(2,6-diisopropylphényl)urée;
- la $N_1$-[2-(7-azaindol-1-yl)hexyl]-$N_2$-(2,6-diisopropylphényl)urée;
- la $N_1$-[1-(3-éthyl-7-azaindol-1-yl)cyclopentylméthyl]-$N_2$-(2,6-diisopropylphényl)urée.
- la $N_1$-[1-(7-azaindol-1-yl)cyclohexylméthyl]-$N_2$-(2,6-diisopropylphényl)urée;
- la $N_1$-[1-(6-chloro-3-phényl-7-azaindol-1-yl)cyclopentylméthyl]-$N_2$-(2,6-diisopropylphényl)urée;
- la $N_1$-[1-(3-éthyl-4-azaindol-1-yl)cyclopentylméthyl]-$N_2$-(2,6-diisopropylphényl)urée;
- la $N_1$-[4-(7-azaindol-1-yl)pyrane-4-ylméthyl]-$N_2$-(2,6-diisopropylphényl)urée;
- la $N_1$-[4-(7-azaindol-1-yl)pent-1-éne-4-yl]-$N_2$-(2,6-diisopropylphényl)urée;
- la $N_1$-[1-(2,3-diméthyl-7-azaindol-1-yl)cyclopentylméthyl]-$N_2$-(2,6-diisopropylphényl)urée;
- la $N_1$-[1-(3-benzyl-7-azaindol-1-yl)cyclopentylméthyl]-$N_2$-(2,6-diisopropylphényl)urée;
- la $N_1$-[1-(3-butyl-7-azaindol-1-yl)cyclopentylméthyl]-$N_2$-(2,6-diisopropylphényl)urée;
- la $N_1$-[1-(3-propyl-7-azaindol-1-yl)cyclopentylméthyl]-$N_2$-(2,6-diisopropylphényl)urée;
- la $N_1$-[1-(3-isopropyl-7-azaindol-1-yl)cyclopentylméthyl]-$N_2$-(2,6-diisopropylphényl)urée;
- la $N_1$-[1-(3-phényl-7-azaindol-1-yl)cyclopentylméthyl]-$N_2$-(2,6-diisopropylphényl)urée;
- la $N_1$-[2-(7-azaindol-1-yl)butyl]-$N_2$-(2,6-diisopropylphényl)urée;
- la $N_1$-[1-(3-méthylthio-7-azaindol-1-yl)cyclopentylméthyl]-$N_2$-(2,6-diisopropylphényl)urée;
- la $N_1$-[1-(5-chloro-3-éthyl-7-azaindol-1-yl)cyclopentylméthyl]-$N_2$-(2,6-diisopropylphényl)urée;
- la $N_1$-[1-(6-azaindol-1-yl)cyclopentylméthyl]-$N_2$-(2,6-diisopropylphényl)urée;
- la $N_1$-[1-(4-méthyl-3-phényl-7-azaindol-1-yl)cyclopentylméthyl]-$N_2$-(2,6-diisopropylphényl)urée;
- la $N_1$-[1-(3-méthyl-7-azaindol-1-yl)cyclopentylméthyl]-$N_2$-(2,6-diisopropylphényl)urée;
- la $N_1$-[1-(3-(4-fluorophényl)-7-azaindol-1-yl)cyclopentylméthyl]-$N_2$-(2,6-diisopropylphényl)urée;
- la $N_1$-[2-(3-phényl-7-azaindol-1-yl)hexyl]-$N_2$-(2,6-diisopropylphényl)urée;
- la $N_1$-[1-(3-(4-méthoxyphényl-7-azaindol-1-yl)cyclopentylméthyl]-$N_2$-(2,6-diisopropylphényl)urée;
- la $N_1$-[1-(3-hexyl-7-azaindol-1-yl)cyclopentylméthyl)-$N_2$-(2,6-diisopropylphényl)urée;
- la $N_1$-[1-(7-azaindol-1-yl)cyclopentylméthyl]-$N_2$-(2,4,6-triméthoxyphényl)urée;
- la $N_1$-[1-(7-azaindol-1-yl)cyclopentylméthyl]-$N_2$-(4,6-diméthoxypyrimidin-5-yl)urée;
- la $N_1$-[1-(7-azaindol-1-yl)cyclopentylméthyl]-$N_2$-(2,6-diéthylphényl)urée;
- la $N_1$-[1-(7-azaindol-1-yl)cyclopentylméthyl]-$N_2$-(2-isopropylphényl)urée;
- la $N_1$-[1-(7-azaindol-1-yl)cyclopentylméthyl]-$N_2$-(2,4-diisopropylphényl)urée;
- la $N_1$-[1-(7-azaindol-1-yl)cyclopentylméthyl]-$N_1$-benzyl-$N_2$-(2,4-difluorophényl)urée;
- la $N_1$-[1-(3-bromo-7-azaindol-1-yl)cyclopentylméthyl]-$N_2$-(2,6-diisopropylphényl)urée;
- la $N_1$-[1-(3-chloro-7-azaindol-1-yl)cyclopentylméthyl]-$N_2$-(2,6-diisopropylphényl)urée;
- la $N_1$-[1-(3-éthényl-7-azaindol-1-yl)cyclopentylméthyl]-$N_2$-(2,6-diisopropylphényl)urée;
- le chlorhydrate de $N_1$-[(7-azaindol-1-yl)cyclopentylméthyl]-$N_2$-(2,6-diisopropylphényl)urée;
- la $N_1$-[1-(7-azaindol-1-yl)cyclopentylméthyl]-$N_1$-benzyl-$N_2$-(2,6-diisopropylphényl)urée;
- le chlorhydrate de $N_1$-[2-(3-éthyl-7-azaindol-1-yl)hexyl)-$N_2$-(2,6-diisopropylphényl)urée.

**4.** Procédés de préparation des composés de formule 1 tels que définis à la revendication 1, caractérisés en ce qu'ils comportent les étapes suivantes,

a) réduction d'un nitrile de formule **6**,

C(R5)(R6)-CN
X1
X2
R4
R2
X3
X4
N
R3
R1
6

b) réaction d'un composé de formule générale **5** avec un composé halogéné $R_8$-X ou bien avec un aldéhyde $R_{10}$-CHO ou un chlorure d'acide $R_{10}$-COCl ou un anhydride d'acide $(R_{10}CO)_2O$ suivie d'une réduction,

C(R5)(R6)-CH(R7)-NH2
X1
X2
R4
R2
X3
X4
N
R3
5
R1

c) la réaction d'un composé de formule générale **4**, avec le phosgène et une amine $R_9NH_2$, ou avec un isocyanate $R_9NCO$,ou avec un trichloroacétamide $Cl_3C$-CONH-$R_9$ ou avec un phénoxycarbamate $C_6H_5$-O-CONH-$R_9$ dans lesquelles $R_1$ à $R_9$ et $X_1$ à $X_4$ ont les mêmes significations que dans la revendication 1 et $R_{10}$ représente un radical alkyle en $C_1$ à $C_{11}$ ou phényle pouvant porter un à trois substituants choisis parmi halogène et les radicaux alkyle, alkoxy et alkylthio en $C_1$ à $C_4$.

C(R5)(R6)-CH(R7)-NH(R8)
X1
X2
R4
R2
X3
X4
N
R3
R1
4

**5.** Composés intermédiaires dans la préparation des composés de formule générale **1** tels que définis à la revendication 1, représentés par les formules générales **4** et **8**,

$C(R_5)(R_6)-CH(R_7)-NH(R_8)$
$X_1$
$X_2$
$R_4$
$R_2$
$X_3$
$X_4$
N
$R_3$
$R_1$
4
$CH(R'_5)CH_2NO_2$
$X_1$
$X_2$
$R_4$
$R_2$
$X_3$
$X_4$
N
$R_3$
$R_1$
8

dans lesquelles, les substituants $R_1$ à $R_7$ et $X_1$ à $X_4$ ont les significations définies dans la revendication 1, $R'_5$ représente un radical phényle pouvant porter des substituants halogène, alkyle, alkoxy, alkylthio en $C_1$-$C_4$ et $R_8$ a les significations définies dans la revendication 1 à l'exception de l'hydrogène.

**6.** Compositions pharmaceutiques caractérisées en ce qu'elles comportent une quantité efficace d'au moins un composé de formule **1** tel que défini à la revendication 1 en mélange à un excipient pharmaceutiquement acceptable.

**7.** Médicaments, notamment à activités hypolipidémiante et anti-athéroscléreuse, selon la revendication 6, sous forme d'unité de dosage dans laquelle chaque unité de dosage contient de 10 à 500 mg de principe actif en mélange à un excipient pharmaceutiquement acceptable.

## Claims

**1.** Compounds of the formula 1

$C(R_5)(R_6)-CH(R_7)-N(R_8)-CO-NH-R_9$
4
$X_1$
5
$X_2$
3
$R_4$
$R_2$
6
$X_3$
$X_4$
2
N
$R_3$
7
1
$R_1$
1

in which
one of the groups $X_1$ to $X_4$ designates a nitrogen atom and the others represent a CH radical;
$R_1$ and $R_2$, which may be situated at position 1, 2 or 3 of the azaindole ring, each represent a hydrogen atom, a linear or branched $C_1$ to $C_{12}$ alkyl radical, a $C_2$ to $C_6$ alkenyl radical, a $C_3$ to $C_8$ cycloalkyl or cycloalkenyl radical, a $C_1$ to $C_6$ alkylthio radical or, when they are at position 2 or 3 of azaindole, a halogen atom; phenyl or benzyl, unsubstituted or having one to three substituents selected from halogen and $C_1$ to $C_4$ alkyl, alkoxy and alkylthio radicals;
$R_3$ and $R_4$, which may be situated on the vertices 4, 5, 6 or 7 of the azaindole ring provided that they have a carbon atom, each represent a hydrogen or halogen atom, $C_1$ to $C_6$ alkyl, alkoxy or alkylthio or $C_3$ to $C_8$ cycloalkyl;
the group $-C(R_5)(R_6)-CH(R_7)-N(R_8)-CO-NH-R_9$ may be attached to the vertices 1, 2 or 3 of the azaindole ring, given that when the nitrogen atom at position 1 of the azaindole ring is not substituted by one of the groups $R_1$, $R_2$ or $-C(R_5)(R_6)-CH(R_7)-N(R_8)CONHR_9$, it has a hydrogen atom;
$R_5$ and $R_6$ each represent a hydrogen atom, a $C_1$ to $C_{12}$ alkyl radical, a $C_2$ to $C_6$ alkenyl radical, a $C_3$ to $C_6$ cycloalkyl or cycloalkenyl radical, a $C_2$ to $C_{12}$ alkoxyalkyl or alkylthioalkyl radical or

$R_5$ and $R_6$ together form an alkylene chain of the formula $-(CH_2)_p-$ optionally substituted by one or two $C_1$ to $C_4$ alkyl radicals and in which p can assume the values 3 to 7, or an alkyloxyalkylene chain of the formula $-(CH_2)_x-O-(CH_2)_y-$, in which x and y can each assume the values 1 or 2;
$R_7$ designates a hydrogen atom, a $C_1$ to $C_6$ alkyl radical or a $C_3$ to $C_8$ cycloalkyl radical;
one of the substituents $R_5$ to $R_7$ may also represent a phenyl or benzyl radical, unsubstituted or having one to three substituents selected from halogen and $C_1$ to $C_4$ alkyl, alkoxy and alkylthio radicals;
$R_8$ designates a hydrogen atom, a linear or branched $C_1$ to $C_{12}$ alkyl radical, a $C_3$ to $C_8$ cycloalkyl radical or a benzyl radical, unsubstituted or having 1 to 3 substituents selected from halogen and $C_1$ to $C_4$ alkyl, alkoxy or alkylthio radicals;
$R_9$ designates a phenyl radical, unsubstituted or having 1 to 3 substituents selected from halogen and $C_1$ to $C_4$ alkyl, alkoxy and alkylthio radicals; 1- or 2-naphthyl or a heterocyclic radical with 5 or 6 members and one or two hetero atoms selected from sulphur, oxygen or nitrogen, optionally fused with a benzene ring and optionally substituted by one or two halogen atoms or $C_1$ to $C_4$ alkyl or alkoxy radicals.

**2.** Compounds according to claim 1 satisfying formula 1, in which
$R_1$ and $R_2$ independently represent hydrogen or halogen atoms or alkyl, phenyl or benzyl radicals, $R_3$ and $R_4$ represent hydrogen or halogen atoms or $C_1$ to $C_6$ alkyl or alkoxy radicals, $R_5$ and $R_6$ represent hydrogen atoms, $C_1$ to $C_6$ alkyl radicals or $C_2$ to $C_6$ alkenyl radicals or together form a $C_4$ alkylene chain, $R_7$ represents a hydrogen atom, $R_8$ represents a hydrogen atom or a $C_1$ to $C_8$ alkyl radical and $R_9$ designates a phenyl group, unsubstituted or having one to three substituents selected from halogen and $C_1$ to $C_4$ alkyl and alkoxy radicals.

**3.** Compounds selected from the group comprising:
- $N_1$-[1-(7-azaindol-1-yl)cyclopentylmethyl]-$N_2$-(2,6-diisopropylphenyl)urea;
- $N_1$-[2-(7-azaindol-1-yl)-2-allylpent-4-enyl)]-$N_2$-(2,6-diisopropylphenyl)urea;
- $N_1$-[2-(7-azaindol-1-yl)-2-allylpent-4-enyl)]-$N_2$-(4-fluoro-2-methylphenyl)urea;
- $N_1$-[2-(7-azaindol-1-yl)-2-phenylethyl)]-$N_2$-(2,6-diisopropylphenyl)urea;
- $N_1$-[2-(7-azaindol-1-yl)-2-methylpropyl)]-$N_2$-(2,6-diisopropylphenyl)urea;
- $N_1$-[1-methyl-7-azaindol-3-yl)cyclopentylmethyl]-$N_2$-(2,6-diisopropylphenyl)urea;
- $N_1$-[1-(5-azaindol-1-yl)cyclopentylmethyl]-$N_2$-(2,6-diisopropylphenyl)urea;
- $N_1$-[2-(7-azaindol-1-yl)hexyl]-$N_2$-(2,6-diisopropylphenyl)urea;
- $N_1$-[1-(3-ethyl-7-azaindol-1-yl)cyclopentylmethyl]-$N_2$-(2,6-diisopropylphenyl)urea;
- $N_1$-[1-(7-azaindol-1-yl)cyclohexylmethyl]-$N_2$-(2,6-diisopropylphenyl)urea;
- $N_1$-[1-(6-chloro-3-phenyl-7-azaindol-1-yl)cyclopentylmethyl]-$N_2$-(2,6-diisopropylphenyl)urea;
- $N_1$-[1-(3-ethyl-4-azaindol-1-yl)cyclopentylmethyl]-$N_2$-(2,6-diisopropylphenyl)urea;
- $N_1$-[4-(7-azaindol-1-yl)pyrane-4-ylmethyl]-$N_2$-(2,6-diisopropylphenyl)urea;
- $N_1$-[4-(7-azaindol-1-yl)pent-1-ene-4-yl]-$N_2$-(2,6-diisopropylphenyl)urea;
- $N_1$-[1-(2,3-dimethyl-7-azaindol-1-yl)cyclopentylmethyl]-$N_2$-(2,6-diisopropylphenyl)urea;
- $N_1$-[1-(3-benzyl-7-azaindol-1-yl)cyclopentylmethyl]-$N_2$-(2,6-diisopropylphenyl)urea;
- $N_1$-[1-(3-butyl-7-azaindol-1-yl)cyclopentylmethyl]-$N_2$-(2,6-diisopropylphenyl)urea;
- $N_1$-[1-(3-propyl-7-azaindol-1-yl)cyclopentylmethyl]-$N_2$-(2,6-diisopropylphenyl)urea;
- $N_1$-[1-(3-isopropyl-7-azaindol-1-yl)cyclopentylmethyl]-$N_2$-(2,6-diisopropylphenyl)urea;
- $N_1$-[1-(3-phenyl-7-azaindol-1-yl)cyclopentylmethyl]-$N_2$-(2,6-diisopropylphenyl)urea;
- $N_1$-[2-(7-azaindol-1-yl)butyl]-$N_2$-(2,6-diisopropylphenyl)urea;
- $N_1$-[1-(3-methylthio-7-azaindol-1-yl)cyclopentylmethyl]-$N_2$-(2,6-diisopropylphenyl)urea;
- $N_1$-[1-(5-chloro-3-ethyl-7-azaindol-1-yl)cyclopentylmethyl]-$N_2$-(2,6-diisopropylphenyl)urea;
- $N_1$-[1-(6-azaindol-1-yl)cyclopentylmethyl]-$N_2$-(2,6-diisopropylphenyl)urea;
- $N_1$-[1-(4-methyl-3-phenyl-7-azaindol-1-yl)cyclopentylmethyl]-$N_2$-(2,6-diisopropylphenyl)urea;
- $N_1$-[1-(3-methyl-7-azaindol-1-yl)cyclopentylmethyl]-$N_2$-(2,6-diisopropylphenyl)urea;
- $N_1$-[1-(3-(4-fluorophenyl)-7-azaindol-1-yl)cyclopentylmethyl]-$N_2$-(2,6-diisopropylphenyl)urea;
- $N_1$-[2-(3-phenyl-7-azaindol-1-yl)hexyl]-$N_2$-(2,6-diisopropylphenyl)urea;
- $N_1$-[1-(3-(4-methoxyphenyl-7-azaindol-1-yl)cyclopentylmethyl]-$N_2$-(2,6-diisopropylphenyl)urea;
- $N_1$-[1-(3-hexyl-7-azaindol-1-yl)cyclopentylmethyl]-$N_2$-(2,6-diisopropylphenyl)urea;
- $N_1$-[1-(7-azaindol-1-yl)cyclopentylmethyl]-$N_2$-(2,4,6-trimethoxyphenyl)urea;
- $N_1$-[1-(7-azaindol-1-yl)cyclopentylmethyl]-$N_2$-(4,6-dimethoxypyrimidin-5-yl)urea;
- $N_1$-[1-(7-azaindol-1-yl)cyclopentylmethyl]-$N_2$-(2,6-diethylphenyl)urea;
- $N_1$-[1-(7-azaindol-1-yl)cyclopentylmethyl]-$N_2$-(2-isopropylphenyl)urea;

- $N_1$-[1-(7-azaindol-1-yl)cyclopentylmethyl]-$N_2$-(2,4-diisopropylphenyl)urea;
- $N_1$-[1-(7-azaindol-1-yl)cyclopentylmethyl]-$N_1$-benzyl-$N_2$-(2,4-difluorophenyl)urea;
- $N_1$-[1-(3-bromo-7-azaindol-1-yl)cyclopentylmethyl]-$N_2$-(2,6-diisopropylphenyl)urea;
- $N_1$-[1-(3-chloro-7-azaindol-1-yl)cyclopentylmethyl]-$N_2$-(2,6-diisopropylphenyl)urea;
- $N_1$-[1-(3-ethenyl-7-azaindol-1-yl)cyclopentylmethyl]-$N_2$-(2,6-diisopropylphenyl)urea;
- $N_1$-[(7-azaindol-1-yl)cyclopentylmethyl]-$N_2$-(2,6-diisopropylphenyl)urea hydrochloride;
- $N_1$-[1-(7-azaindol-1-yl)cyclopentylmethyl]-$N_1$-benzyl-$N_2$-(2,6-diisopropylphenyl)urea;
- $N_1$-[2-(3-ethyl-7-azaindol-1-yl)hexyl]-$N_2$-(2,6-diisopropylphenyl)urea hydrochloride.

**4.** Processes for the preparation of the compounds of formula 1 as defined in claim 1, characterised in that they comprise the following steps:

a) reduction of a nitrile of formula 6,

C(R5)(R6)-CN
X1
X2
R4
X3
X4
N
R2
R3
R1
6

b) reaction of a compound of general formula 5 with a halogenated compound $R_8$-X or with an aldehyde $R_{10}$-CHO or an acid chloride $R_{10}$-COCl or an acid anhydride $(R_{10}CO)_2O$ followed by a reduction,

C(R5)(R6)-CH(R7)-NH2
X1
X2
R4
X3
X4
N
R2
R3
R1
5

c) the reaction of a compound of general formula 4 with phosgene and an amine $R_9NH_2$ or with an isocyanate $R_9NCO$ or with a trichloroacetamide $Cl_3C$-CONH-$R_9$ or with a phenoxycarbamate $C_6H_5$-O-CONH-$R_9$, in which $R_1$ to $R_9$ and $X_1$ to $X_4$ have the same meanings as in claim 1 and $R_{10}$ represents a $C_1$ to $C_{11}$ alkyl radical or phenyl which may have one to three substituents selected from halogen and $C_1$ to $C_4$ alkyl, alkoxy and alkylthio radicals.

$$C(R_5)(R_6)\text{-}CH(R_7)\text{-}NH(R_8)$$

4

5. Intermediate compounds in the preparation of the compounds of general formula 1 as defined in claim 1, represented by the general formulae 4 and 8,

$$C(R_5)(R_6)\text{-}CH(R_7)\text{-}NH(R_8)$$

4

$$CH(R'_5)CH_2NO_2$$

8

in which the substituents $R_1$ to $R_7$ and $X_1$ to $X_4$ have the meanings defined in claim 1, $R'_5$ represents a phenyl radical which may have the substituents halogen, $C_1$-$C_4$ alkyl, alkoxy or alkylthio and $R_8$ has the meanings defined in claim 1, with the exception of hydrogen.

6. Pharmaceutical compositions characterized in that they include an effective quantity of at least one compound of formula 1 as defined in claim 1 mixed with a pharmaceutically acceptable carrier.

7. Medicines, in particular displaying hypolipidaemic and antiatherosclerotic activity according to claim 6 in the form of a dosage unit in which each dosage unit contains from 10 to 500 mg of active principle mixed with a pharmaceutically acceptable carrier.

**Patentansprüche**

1. Verbindungen der Formel 1

$$C(R_5)(R_6)\text{-}CH(R_7)\text{-}N(R_8)\text{-}CO\text{-}NH\text{-}R_9$$

1

in der

eine der Gruppen $X_1$ bis $X_4$ ein Stickstoffatom und die anderen eine CH-Gruppe bedeuten:

$R_1$ und $R_2$, die In der 1-, 2- oder 3-Stellung des Azaindolrings stehen können, jeweils ein Wasserstoffatom, eine geradkettige oder verzweigte $C_1$-$C_{12}$-Alkylgruppe, $C_2$-$C_6$-Alkenylgruppe, $C_3$-$C_8$-Cycloalkylgruppe, $C_3$-$C_8$-Cycloalkenylgruppe, $C_1$-$C_6$-Alkylthiogruppe oder, wenn sie in der 2- oder 3-Stellung des Azaindolrings stehen, ein Halogenatom: oder eine Phenyl- oder Benzylgruppe, die nichtsubstituiert ist oder einen bis drei Substituenten ausgewählt aus Halogenatomen, $C_1$-$C_4$-Alkylgruppen, $C_1$-$C_4$-Alkoxygruppen und $C_1$-$C_4$-Alkylthiogruppen trägt, darstellen;

$R_3$ und $R_4$, die in der 4-, 5-, 6- oder 7-Stellung des Azaindolrings stehen können, mit der Maßgabe, daß diese ein Kohlenstoffatom bedeuten, jeweils ein Wasserstoffatom, ein Halogenatom, eine $C_1$-$C_6$-Alkylgruppe, eine $C_1$ -$C_6$-Alkoxygruppe, eine $C_1$-$C_6$-Alkylthiogruppe oder eine $C_3$-$C_8$-Cycloalkylgruppe darstellen;

die Gruppe -C($R_5$)($R_6$)-CH($R_7$)-N($R_8$)-CO-NH-$R_9$ an die 1-, 2- oder 3-Stellung des Azaindolrings gebunden sein kann, mit der Maßgabe, daß, wenn das Stickstoffatom in der 1-Stellung des Azaindolrings nicht durch eine der Gruppe $R_1$, $R_2$ oder -C($R_5$)($R_6$)-CH($R_7$)-N($R_8$)-CONH$R_9$ substituiert ist, es ein Wasserstoffatom trägt;

$R_5$ und $R_6$ jeweils ein Wasserstoffatom, eine $C_1$-$C_{12}$-Alkylgruppe, eine $C_2$-$C_6$-Alkenylgruppe, eine $C_3$-$C_8$-Cycloalkylgruppe, eine $C_3$-$C_8$-Cycloalkenylgruppe, eine $C_2$-$C_{12}$-Alkoxyalkylgruppe oder eine $C_2$-$C_{12}$-Alkylthioalkylgruppe darstellen, oder

$R_5$ und $R_6$ gemeinsam eine Alkylenkette der Formel -$(CH_2)_p$- bilden, die gegebenenfalls durch eine oder zwei $C_1$-$C_4$-Alkylgruppen substituiert ist und worin p Werte von 3 bis 7 annehmen kann, oder eine Alkyloxyalkylengruppe der Formel -$(CH_2)_x$-O-$(CH_2)_y$- bilden, worin x und y jeweils die Werte 1 oder 2 besitzen können;

$R_7$ ein Wasserstoffatom, eine $C_1$-$C_6$-Alkylgruppe oder eine $C_3$-$C_8$-Cycloalkylgruppe darstellt;

einer der Substituenten $R_5$ bis $R_7$ zusätzlich eine Phenyl- oder Benzylgruppe darstellen kann, die nichtsubstituiert ist oder einen bis drei Substituenten ausgewählt aus Halogenatomen und $C_1$-$C_4$-Alkylgruppen, $C_1$-$C_4$-Alkoxygruppen und $C_1$-$C_4$-Alkylthiogruppen tragen kann;

$R_8$ ein Wasserstoffatom, eine geradkettige oder verzweigte $C_1$-$C_{12}$-Alkylgruppe, eine $C_3$-$C_8$-Cycloalkylgruppe oder eine Benzylgruppe, die nichtsubstituiert ist oder 1 bis 3 Sbustituenten ausgewählt aus Halogenatomen und $C_1$-$C_4$-Alkylgruppen, $C_1$-$C_4$-Alkoxygruppen oder $C_1$-$C_4$-Alkylthiogruppen trägt, bedeutet;

$R_9$ eine Phenylgruppe, die nichtsubstituiert ist oder 1 bis 3 Substituenten ausgewählt aus Halogenatomen und $C_1$-$C_4$-Alkylgruppen, $C_1$-$C_4$-Alkoxygruppen und $C_1$-$C_4$-Alkylthiogruppen trägt; eine 1-Naphthyl- oder 2-Naphthylgruppe oder eine heterocyclische Gruppe mit 5 oder 6 Kettengliedern und 1 oder 2 Heteroatomen ausgewählt aus Schwefel, Sauerstoff oder Stickstoff, die gegebenenfalls mit einem Benzolring kondensiert ist und gegebenenfalls durch eines oder zwei Halogenatome oder $C_1$-$C_4$-Alkyl- oder $C_1$-$C_4$-Alkoxygruppen substituiert ist, bedeutet.

**2.** Verbindungen nach Anspruch 1 der Formel 1, in der $R_1$ und $R_2$ unabhängig voneinander Wasserstoffatome oder Halogenatome, Alkylgruppen, Phenylgruppen oder Benzylgruppen, $R_3$ und $R_4$ Wasserstoffatome oder Halogenatome oder $C_1$-$C_6$-Alkylgruppen oder $C_1$-$C_6$-Alkoxygruppen, $R_5$ und $R_6$ Wasserstoffatome, $C_1$-$C_6$-Alkylgruppen, $C_2$-$C_6$-Alkenylgruppen oder gemeinsam eine $C_4$-Alkylenkette, $R_7$ ein Wasserstoffatom, $R_8$ ein Wasserstoffatom oder eine $C_1$-$C_8$-Alkylgruppe und $R_9$ eine Phenylgruppe, die nichtsubstituiert ist oder 1 bis 3 Substituenten ausgewählt aus Halogenatomen und $C_1$-$C_4$-Alkylgruppen und $C_1$-$C_4$-Alkoxygruppen trägt, bedeuten.

**3.** Verbindungen ausgewählt aus der folgenden Gruppe:

- $N_1$-[1-(7-Azaindol-1-yl)cyclopentylmethyl]-$N_2$-(2,6-diisopropylphenyl)-harnstoff;
- $N_1$-[2-(7-Azaindol-1-yl)-2-allylpenten-4-yl]-$N_2$-(2,6-diisopropylphenyl)-harnstoff;
- $N_1$-[2-(7-Azaindol-1-yl)-2-allylpenten-4-yl]-$N_2$-(4-fluor-2-methylphenyl)-harnstoff;
- $N_1$-[2-(7-Azaindol-1-yl)-2-phenylethyl]-$N_2$-[2,6-diisopropylphenyl)-harnstoff;
- $N_1$-[2-(7-Azaindol-1-yl)-2-methylpropyl]-$N_2$-(2,6-diisopropylphenyl)-harnstoff;
- $N_1$-[1-(Methyl-7-azaindol-3-yl)-cyclopentylmethyl]-$N_2$-(2,6-diisopropylphenyl)-harnstoff;
- $N_1$-[1-(5-Azaindol-1-yl)-cyclopentylmethyl]-$N_2$-(2,6-diisopropylphenyl)-harnstoff;
- $N_1$-[2-(7-Azaindol-1-yl)-hexyl]-$N_2$-(2,6-diisopropylphenyl)-harnstoff;
- $N_1$-[1-(3-Ethyl-7-azaindol-1-yl)-cyclopentylmethyl]-$N_2$-(2,6-diisopropylphenyl)-harnstoff;
- $N_1$-[1-(7-Azaindol-1-yl)-cyclohexylmethyl]-$N_2$-(2,6-diisopropylphenyl)-harnstoff;
- $N_1$-[1-(6-Chlor-3-phenyl-7-azaindol-1-yl)-cyclopentylmethyl]-$N_2$-(2,6-diisopropylphenyl)-harnstoff;
- $N_1$-[1-(3-Ethyl-4-azaindol-1-yl)-cyclopentylmethyl]-$N_2$-(2,6-diisopropylphenyl)-harnstoff;
- $N_1$-[4-(7-Azaindol-1-yl)-pyran-4-yl-methyl]-$N_2$-(2,6-diisopropylphenyl)-harnstoff;
- $N_1$-[4-(7-Azaindol-1-yl)-pent-1-en-4-yl]-$N_2$-(2,6-diisopropylphenyl)-harnstoff;

- $N_1$-[1-(2,3-Dimethyl-7-azaindol-1-yl)-cyclopentylmethyl]-$N_2$-(2,6-diisopropylphenyl)-harnstoff;
- $N_1$-[1-(3-Benzyl-7-azaindol-1-yl)-cyclopentylmethyl]-$N_2$-(2,6-diisopropylphenyl)-harnstoff;
- $N_1$-[1-(3-Butyl-7-azaindol-1-yl)-cyclopentylmethyl]-$N_2$-(2,6-diisopropylphenyl)-harnstoff;
- $N_1$-[1-(3-Propyl-7-azaindol-1-yl)-cyclopentylmethyl]-$N_2$-(2,6-diisopropylphenyl)-harnstoff;
- $N_1$-[1-(3-Isopropyl-7-azaindol-1-yl)-cyclopentylmethyl]-$N_2$-(2,6-diisopropylphenyl)harnstoff;
- $N_1$-[1-(3-Phenyl-7-azaindol-1-yl)-cyclopentylmethyl]-$N_2$-(2,6-diisopropylphenyl)-harnstoff;
- $N_1$-[2-(7-Azaindol-1-yl)-butyl]-$N_2$-(2,6-diisopropylphenyl)-harnstoff;
- $N_1$-[1-(3-Methylthio-7-azaindol-1-yl)-cyclopentylmethyl]-$N_2$-(2,6-diisopropylphenyl)-harnstoff:
- $N_1$-[1-(5-Chlor-3-ethyl-7-azaindol-1-yl)-cyclopentylmethyl]-$N_2$-(2,6-diisopropylphenyl)-harnstoff;
- $N_1$-[1-(6-Azaindol-1-yl)-cyclopentylmethyl]-$N_2$-(2,6-diisopropylphenyl)-harnstoff;
- $N_1$-[1-(4-Methyl-3-phenyl-7-azaindol-1-yl)-cyclopentylmethyl]-$N_2$-(2,6-diisopropylphenyl)-harnstoff;
- $N_1$-[1-(3-Methyl-7-azaindol-1-yl)-cyclopentylmethyl]-$N_2$-(2,6-diisopropylphenyl)-harnstoff;
- $N_1$-[1-(3-(4-Fluorphenyl)-7-azaindol-1-yl)-cyclopentylmethyl]-$N_2$-(2,6-diisopropylphenyl)-harnstoff;
- $N_1$-[2-(3-Phenyl-7-azaindol-1-yl)-hexyl]-$N_2$-(2,6-diisopropylphenyl)-harnstoff;
- $N_1$-[1-(3-(4-Methoxyphenyl)-7-azaindol-1-yl)-cyclopentylmethyl]-$N_2$-(2,6-diisopropylphenyl)-harnstoff;
- $N_1$-[1-(3-Hexyl-7-azaindol-1-yl)-cyclopentylmethyl]-$N_2$-(2,6-diisopropylphenyl)-harnstoff;
- $N_1$-[1-(7-Azaindol-1-yl)-cyclopentylmethyl]-$N_2$-(2,4,6-trimethoxyphenyl)-harnstoff;
- $N_1$-[1-(7-Azaindol-1-yl)-cyclopentylmethyl]-$N_2$-(4,6-dimethoxypyrimidin-5-yl)-harnstoff;
- $N_1$-[1-(7-Azaindol-1-yl)-cyclopentylmethyl]-$N_2$-(2,6-diethylphenyl)-harnstoff;
- $N_1$-[1-(7-Azaindol-1-yl)-cyclopentylmethyl]-$N_2$-(2-isopropylphenyl)-harnstoff;
- $N_1$-[1-(7-Azaindol-1-yl)-cyclopentylmethyl]-$N_2$-(2,4-diisopropylphenyl)-harnstoff;
- $N_1$-[1-(7-Azaindol-1-yl)-cyclopentylmethyl]-$N_1$-benzyl-$N_2$-(2,4-difluorphenyl)-harnstoff;
- $N_1$-[1-(3-Brom-7-azaindol-1-yl)-cyclopentylmethyl]-$N_2$-(2,6-diisopropylphenyl)-harnstoff;
- $N_1$-[1-(3-Chlor-7-azaindol-1-yl)-cyclopentylmethyl]-$N_2$-(2,6-diisopropylphenyl)-harnstoff;
- $N_1$-[1-(3-Ethenyl-7-azaindol-1-yl)-cyclopentylmethyl]-$N_2$-(2,6-diisopropylphenyl)-harnstoff;
- Hydrochlorid von $N_1$-[(7-Azaindol-1-yl)-cyclopentylmethyl]-$N_2$-(2,6-diisopropylphenyl)-harnstoff;
- $N_1$-[1-(7-Azaindol-1-yl)-cyclopentylmethyl]-$N_1$-benzyl-$N_2$-(2,6-diisopropylphenyl)-harnstoff:
- Hydrochlorid von $N_1$-[2-(3-Ethyl-7-azaindol-1-yl)-hexyl]-$N_2$-(2,6-diisopropylphenyl)-harnstoff.

**4.** Verfahren zur Herstellung der Verbindungen der Formel 1, wie sie in Anspruch 1 definiert ist, **dadurch gekennzeichnet**, daß sie die folgenden Stufen umfassen:

a) Reduktion eines Nitrils der Formel 6

b) Umsetzung einer Verbindung der allgemeinen Formel 5

mit einer Halogenverbindung $R_8$-X oder einem Aldehyd $R_{10}$-CHO oder einem Säurechlorid $R_{10}$-COCl oder einem Säureanhydrid $(R_{10}CO)_2O$, gefolgt von einer Reduktion,

c) die Reaktion einer Verbindung der allgemeinen Formel 4

4

mit Phosgen und einem Amin $R_9NH_2$ oder einem Isocyanat $R_9NCO$ oder einem Trichloracetamid $Cl_3C$-CONH-$R_9$ oder einem Phenoxycarbamat $C_6H_5$-O-CONH-$R_9$, worin $R_1$ bis $R_9$ und $X_1$ bis $X_4$ die in Anspruch 1 angegebenen Bedeutungen besitzen und $R_{10}$ eine $C_1$-$C_{11}$-Alkylgruppe oder eine Phenylgruppe, die einen bis drei Substituenten, ausgewählt aus Halogenatomen und $C_1$-$C_4$-Alkylgruppen, $C_1$-$C_4$-Alkoxygruppen und $C_1$-$C_4$-Alkylthiogruppen tragen kann, bedeuten.

**5.** Zwischenprodukte für die Herstellung der Verbindungen der allgemeinen Formel 1, wie sie in Anspruch 1 definiert ist, der allgemeinen Formeln 4 und 8

4

8

worin die Substituenten $R_1$ bis $R_7$ und $X_1$ bis $X_4$ die in Anspruch 1 angegebenen Bedeutungen besitzen, $R'_5$ eine Phenylgruppe darstellt, die Halogen-, $C_1$-$C_4$-Alkyl-, $C_1$-$C_4$-Alkoxy- oder $C_1$-$C_4$-Alkylthio-Substituenten tragen kann, und $R_8$ die in Anspruch 1 angegebenen Bedeutungen besitzt mit Ausnahme von Wasserstoff.

**6.** Pharmazeutische Zubereitungen, **dadurch gekennzeichnet**, daß sie eine wirksame Menge mindestens einer Verbindung der Formel 1, wie sie in Anspruch 1 definiert worden ist, in Mischung mit einem pharmazeutisch annehmbaren Trägermaterial enthalten.

**7.** Arzneimittel mit insbesondere hypolipidämischer und anti-arteriosklerotischer Wirkung nach Anspruch 6 in Form von Dosiseinheiten, in der jede Dosiseinheit 10 bis 500 mg des Wirkstoffs in Mischung mit einem pharmazeutisch annehmbaren Trägermaterial enthält.